# EUROPEAN PATENT APPLICATION

(11) **EP 4 725 406 A1**
(43) Date of publication of application: **15.04.2026**
(21) Application number: 24884314.6
(22) Date of filing: 24.09.2024
(51) Int. Cl.: A61B 5/0205

(54) **BODY TEMPERATURE MEASUREMENT METHOD, WEARABLE DEVICE, AND STORAGE MEDIUM**

(30) Priority: 31.10.2023 CN 202311442934
(71) Applicant: Huawei Technologies Co., Ltd., Shenzhen, Guangdong 518129 (CN)
(72) Inventor: ZHAO, Shuai, Shenzhen, Guangdong 518129 (CN); REN, Huichao, Shenzhen, Guangdong 518129 (CN); CAO, Yu, Shenzhen, Guangdong 518129 (CN); YUAN, Jili, Shenzhen, Guangdong 518129 (CN)
(74) Representative: Gill Jennings & Every LLP
(86) International application number: PCT/CN2024/120862
(87) International publication number: WO 2025/092307

(57) **Abstract**

A body temperature measurement method, a wearable device, and a storage medium are provided. The method includes: When a wearable device (100) is worn on a human body, the wearable device (100) obtains a first measurement value obtained by a first temperature sensor by performing body temperature measurement on a first measured part of the human body at a first measurement moment, and obtains an ambient temperature and a first heart rate value of the human body at the first measurement moment; inputs the first measurement value, the ambient temperature, and the first heart rate value into a body temperature model, to obtain a first body temperature value of the human body; and then displays the first body temperature value. A structure parameter of the body temperature model is a preset value, or is generated by performing model training based on standard body temperature values and heart rate values of the human body in historical duration. With reference to data such as the first heart rate value of the human body and the ambient temperature at the first measurement moment, the first measurement value is calibrated by using the body temperature model, so that a more accurate body temperature can be obtained, thereby improving accuracy of body temperature measurement performed by the wearable device.

## Description

### CROSS-REFERENCE TO RELATED APPLICATIONS

This application claims priority to Chinese Patent Application No. 202311442934.6, filed with the China National Intellectual Property Administration on October 31, 2023 and entitled "BODY TEMPERATURE MEASUREMENT METHOD, WEARABLE DEVICE, AND STORAGE MEDIUM", which is incorporated herein by reference in its entirety.

### TECHNICAL FIELD

This application relates to the field of electronic technologies, and in particular, to a body temperature measurement method, a wearable device, and a storage medium.

### BACKGROUND

A body temperature is a temperature necessary for maintaining normal functions of human organs such as the brain, heart, and lungs. In normal cases, the body temperature fluctuates within a range of approximately 36°C to 37.2°C. There are a plurality of factors that affect changes of the body temperature, such as infectious fever and non-infectious fever. Therefore, body temperature detection is a necessary means of health monitoring. Furthermore, a capability of round-the-clock monitoring can be provided through body temperature detection via a wearable device, to provide a timely fever warning and identification of a health problem. A common body temperature measurement method in the conventional technology is that an ear thermometer, a forehead thermometer, or the like is used to measure a core temperature of a human body through infrared measurement on an eardrum, a forehead, or the like of the human body. However, this body temperature measurement method cannot implement convenient, continuous, and unobtrusive monitoring, and requires the human body to actively initiate measurement. In addition, the ear thermometer and the forehead thermometer are usually large in size and are inconvenient to carry. Currently, there is a convenient body temperature measurement method. For example, a smartwatch is worn on a wrist, and the body temperature is inferred by measuring a temperature of a rear case of the smartwatch. However, the temperature that the smartwatch obtains through measurement is a wrist temperature, which differs substantially from a core body temperature because the wrist is a distal part of the human body, and is susceptible to an ambient temperature and external interference, making it impossible to accurately measure the body temperature.

### SUMMARY

Embodiments of this application provide a body temperature measurement method, a wearable device, and a storage medium, to improve accuracy of body temperature measurement performed by the wearable device.

According to a first aspect, this application provides a body temperature measurement method. The method may be performed by a wearable device or a component in the wearable device, and the wearable device includes a first temperature sensor and a heart rate sensor. For example, the method may be performed by the wearable device. In the method, when the wearable device is worn on a human body, the wearable device obtains a first measurement value obtained by the first temperature sensor by performing body temperature measurement on a first measured part of the human body, where the first measurement value corresponds to a first measurement moment; the wearable device obtains an ambient temperature at the first measurement moment and a first heart rate value obtained by the heart rate sensor by performing heart rate measurement on the human body at the first measurement moment; the wearable device inputs the first measurement value, and the ambient temperature and the first heart rate value at the first measurement moment into a body temperature model, to obtain a first body temperature value of the human body, where a structure parameter of the body temperature model is a preset value, or a structure parameter of the body temperature model is generated by performing model training based on standard body temperature values and heart rate values of the human body in historical duration; and the wearable device displays the first body temperature value.

Based on the method, when the wearable device is worn on the human body, the first temperature sensor may perform body temperature measurement on the first measured part to obtain the first measurement value at the first measurement moment, and then the first measurement value is calibrated by using the body temperature model with reference to data such as the first heart rate value of the human body and the ambient temperature at the first measurement moment, so that a more accurate body temperature can be obtained, thereby improving accuracy of body temperature measurement performed by the wearable device.

In a possible implementation, the structure parameter of the body temperature model is a preset value, and after obtaining the first body temperature value of the human body, the method further includes: displaying first prompt information if the first body temperature value is greater than or equal to a first threshold, where the first prompt information is used to prompt to enter the standard body temperature value. In this implementation, the wearable device may obtain a body temperature value that is of the human body and that is greater than or equal to the first threshold.

In a possible implementation, after the wearable device displays the first prompt information, the wearable device may further obtain at least one entered second measurement moment and the standard body temperature value corresponding to each second measurement moment, obtain a second heart rate value obtained by the heart rate sensor through measurement at each second measurement moment, and then obtain at least one set of first data pairs, where different sets of first data pairs are created based on the standard body temperature values and the second heart rate values that correspond to different second measurement moments; and the wearable device updates the structure parameter of the body temperature model based on the at least one set of first data pairs. In this implementation, a plurality of sets of accurate first data pairs may be obtained, to obtain an updated body temperature model with higher body temperature prediction accuracy.

In a possible implementation, that the wearable device updates the structure parameter of the body temperature model based on the at least one set of first data pairs includes: displaying, by the wearable device, second prompt information, where the second prompt information is used to prompt to update the structure parameter of the body temperature model; receiving a first instruction, where the first instruction instructs the wearable device to update the body temperature model; and updating, in response to the first instruction, the structure parameter of the body temperature model based on the at least one set of first data pairs. In this implementation, the wearable device may provide an update prompt for a user, so that the user timely updates the structure parameter of the body temperature model.

In a possible implementation, before updating the structure parameter of the body temperature model based on the at least one set of first data pairs, the method further includes: determining that a quantity of data pairs in the at least one first data pair reaches a quantity threshold. In this implementation, the wearable device may prompt the user to update only when the quantity of the first data pairs is sufficient, thereby obtaining the structure parameter with high body temperature prediction accuracy.

In a possible implementation, after updating the structure parameter of the body temperature model, the method may further include: updating the structure parameter of the body temperature model when the wearable device creates a first data pair or deletes a created first data pair. The body temperature model can be quickly updated in this manner.

In a possible implementation, the method further includes: if the first body temperature value is greater than or equal to a second threshold, prompting to enable a scheduled temperature measurement reminder function, where the second threshold is greater than the first threshold. In this manner, when a body temperature of the human body is excessively high, the scheduled temperature measurement reminder function is prompted to be enabled, to remind the user to measure the body temperature at regular intervals.

In a possible implementation, the method may further include: receiving a second instruction, where the second instruction instructs the wearable device to enter a preset mode, and the preset mode is a mode in which body temperature measurement is performed on a second measured part of the human body; and after entering the preset mode in response to the second instruction, if it is determined that the wearable device meets a measurement condition corresponding to the preset mode, performing body temperature measurement on the second measured part of the human body by using the first temperature sensor, to obtain a second measurement value of the second measured part at a third measurement moment; and displaying the second measurement value. In this implementation, the wearable device may directly measure the second measured part, to obtain an accurate body temperature value of the human body.

In a possible implementation, the second measured part includes an axilla or a forehead.

In a possible implementation, the wearable device further includes a second temperature sensor and a motion sensor. That the wearable device determines that the wearable device meets the measurement condition corresponding to the preset mode includes: obtaining, by the wearable device, detection data of the motion sensor; obtaining, by the wearable device, a first temperature change rate corresponding to a plurality of third measurement values obtained by the first temperature sensor by performing body temperature measurement on the second measured part for a plurality of times within first preset duration after the wearable device enters the preset mode, and obtaining a second temperature change rate corresponding to a plurality of fourth measurement values obtained by the second temperature sensor by performing body temperature measurement on the second measured part for a plurality of times within the first preset duration after the wearable device enters the preset mode; and if the detection data meets a posture condition corresponding to the preset mode, the first temperature change rate is greater than or equal to a third threshold, and the second temperature change rate is greater than or equal to a fourth threshold, determining that the wearable device meets the measurement condition corresponding to the preset mode. This implementation provides a manner for determining that the wearable device meets the measurement condition corresponding to the preset mode.

In a possible implementation, the method may further include: if it is determined that the wearable device does not meet the measurement condition corresponding to the preset mode, displaying third prompt information, where the third prompt information is used to prompt to adjust a placement posture of the wearable device. In this implementation, when the wearable device does not meet the measurement condition corresponding to the preset mode, the user may be timely reminded to adjust the placement posture of the wearable device.

In a possible implementation, the third measurement moment is a corresponding measurement moment when a fifth measurement value obtained by the first temperature sensor by performing body temperature measurement on the second measured part and a sixth measurement value obtained by the second temperature sensor by performing body temperature measurement on the second measured part meet a preset condition, where the preset condition includes: the fifth measurement value is greater than or equal to a fifth threshold, the sixth measurement value is greater than or equal to the fifth threshold, and a difference between the fifth measurement value and the sixth measurement value is less than or equal to a sixth threshold. In this implementation, it is convenient for the wearable device to determine a temperature measurement completion moment, to obtain a body temperature that is of the second measured part of the human body and that is obtained through measurement in the preset mode.

In a possible implementation, performing body temperature measurement on the second measured part of the human body by using the first temperature sensor and/or the second temperature sensor, to obtain the second measurement value of the second measured part at the third measurement moment includes: using the fifth measurement value or the sixth measurement value as the second measurement value, or using an average value of the fifth measurement value and the sixth measurement value as the second measurement value.

In a possible implementation, the method further includes: obtaining the plurality of third measurement values obtained by the first temperature sensor by performing body temperature measurement on the second measured part for the plurality of times within the first preset duration after the wearable device enters the preset mode and a measurement moment corresponding to each third measurement value, and obtaining the plurality of fourth measurement values obtained by the second temperature sensor by performing body temperature measurement on the second measured part for the plurality of times within the first preset duration after the wearable device enters the preset mode and a measurement moment corresponding to each fourth measurement value; obtaining a plurality of first sampling points and a plurality of second sampling points, where different first sampling points include different third measurement values and corresponding measurement moments, and different second sampling points include different fourth measurement values and corresponding measurement moments; and obtaining the second measurement value through calculation by using a least square method based on a first model, a second model, the plurality of first sampling points, and the plurality of second sampling points, where the first model is used to represent a relationship in which a temperature of the first temperature sensor changes over time within second preset duration, and the second model is used to represent a relationship in which a temperature of the second temperature sensor changes over time within the second preset duration. In this implementation, a steady-state body temperature measurement value of the second measured part is predicted by using an algorithm, so that measurement time consumption can be reduced.

In a possible implementation, after obtaining, by the wearable device, the second measurement value of the second measured part at the third measurement moment, the method may further include: obtaining, by the wearable device, a third heart rate value obtained by the heart rate sensor by performing heart rate measurement on the human body at the third measurement moment; and creating a second data pair based on the second measurement value and the third heart rate value, where the second data pair is used to update the structure parameter of the temperature model. Alternatively, the second measurement value in this implementation may be used as a standard temperature value, and creates one second data pair together with the third heart rate value corresponding to the third measurement moment, so that a quantity of data pairs used to update the structure parameter of the temperature model can be increased.

According to a second aspect, this application further provides an apparatus. The apparatus includes modules/units for performing the method according to any possible design in any one of the foregoing aspects. These modules/units may be implemented by hardware, or may be implemented by hardware executing corresponding software.

According to a third aspect, this application provides a wearable device, including a processor, a memory, a temperature sensor, a heart rate sensor, and a display. The display is configured to display a user interface. The temperature sensor is configured to perform body temperature measurement on a first measured part or a second measured part of a human body. The heart rate sensor is configured to perform heart rate measurement on the human body. The memory is configured to store one or more computer programs. The processor is configured to: execute the one or more computer programs, and perform the method according to any one of the first aspect and the possible designs of the first aspect with reference to data obtained by the temperature sensor through measurement and data obtained by the heart rate sensor through measurement.

In a possible implementation, the wearable device further includes a motion sensor, configured to detect a placement posture of the wearable device.

According to a fourth aspect, this application further provides a readable storage medium. The readable storage medium includes a program, and when the program is run on a wearable device, the wearable device is enabled to perform the method according to any one of the first aspect and the possible designs of the first aspect.

According to a fifth aspect, this application further provides a computer program product. When the computer program product is run on a wearable device, the wearable device is enabled to perform the method according to any one of the first aspect and the possible designs of the first aspect.

### BRIEF DESCRIPTION OF DRAWINGS

FIG. 1 is a diagram of a structure of a wearable device according to an embodiment of this application;
FIG. 2 is a schematic flowchart of a body temperature measurement method according to an embodiment of this application;
FIG. 3 is a diagram of a wearable device according to an embodiment of this application;
FIG. 4 is a diagram in which a wearable device is worn on a wrist of a human body according to an embodiment of this application;
FIG. 5 is a diagram of updating a model according to an embodiment of this application;
FIG. 6A and FIG. 6B are a schematic flowchart of a body temperature measurement method according to an embodiment of this application;
FIG. 7A, FIG. 7B, and FIG. 7C are diagrams of a set of user interfaces according to an embodiment of this application;
FIG. 8 is a diagram of a set of user interfaces according to an embodiment of this application;
FIG. 9A and FIG. 9B are diagrams of a set of user interfaces according to an embodiment of this application;
FIG. 10A and FIG. 10B are diagrams of a set of user interfaces according to an embodiment of this application;
FIG. 11A and FIG. 11B are diagrams of a set of user interfaces according to an embodiment of this application;
FIG. 12 is a schematic flowchart of a body temperature measurement method according to an embodiment of this application;
FIG. 13A, FIG. 13B, and FIG. 13C are diagrams of a set of user interfaces according to an embodiment of this application;
FIG. 14 is a diagram of a temperature change that is of a temperature sensor and that is obtained after a wearable device enters an axillary mode according to an embodiment of this application;
FIG. 15 is a schematic flowchart of a temperature measurement process after a wearable device enters an axillary mode according to an embodiment of this application;
FIG. 16 is a diagram of a temperature measurement posture of a user for measuring a forehead temperature of the user according to an embodiment of this application;
FIG. 17 is a diagram of a temperature measurement posture of a user for measuring a forehead temperature of another person according to an embodiment of this application; and
FIG. 18 is a diagram of a structure of a wearable device according to an embodiment of this application.

### DESCRIPTION OF EMBODIMENTS

The following clearly and completely describes the technical solutions in embodiments of this application with reference to the accompanying drawings in embodiments of this application. Terms used in the following embodiments are merely intended to describe specific embodiments, but are not intended to limit this application. Terms "one", "a", "the", "the foregoing", "this", and "the one" of singular forms used in this specification and the appended claims of this application are also intended to include expressions such as "one or more", unless otherwise specified in the context clearly. It should be further understood that, in embodiments of this application, "one or more" means one, two, or more, and "and/or" describes an association relationship between associated objects, and indicates that three relationships may exist. For example, A and/or B may indicate the following cases: Only A exists, both A and B exist, and only B exists, where A and B may be singular or plural. The character "/" generally indicates an "or" relationship between the associated objects.

Reference to "some embodiments" or the like described in this specification indicates that one or more embodiments of this application include a specific feature, structure, or characteristic described with reference to embodiments. Therefore, statements such as "in some embodiments", "in some other embodiments", and "in other embodiments" that appear at different places in this specification do not necessarily refer to a same embodiment, but mean "one or more but not all embodiments", unless otherwise specifically emphasized in another manner. Terms "include", "comprise", "have", and variants thereof all mean "include but are not limited to", unless otherwise specifically emphasized in another manner.

"A plurality of" in embodiments of this application means two or more. It should be noted that, in the descriptions of embodiments of this application, terms such as "first" and "second" are merely intended for distinction in description, but should not be construed as indicating or implying relative importance or indicating or implying a sequence.

This application provides a body temperature measurement method, a wearable device, and a storage medium, to improve accuracy of body temperature measurement performed by the wearable device. The method and the wearable device are based on a same technical concept. Because problem-resolving principles of the method and the wearable device are similar, mutual reference may be made to implementation of the wearable device and the method. Repeated parts are not described.

In the solutions provided in embodiments of this application, the wearable device includes a device (for example, a processor, an application processor, a graphics processing unit, or another processor) that can implement a data processing function. An example embodiment of the wearable device includes but is not limited to a device provided with iOS^{®}, Android^{®}, Microsoft^{®}, or another operating system. The wearable device may alternatively be another portable device, for example, a laptop computer (laptop) with a touch-sensitive surface (for example, a touch panel). A specific form of the wearable device is not limited in embodiments of this application.

For example, the wearable device may be a smartwatch, a smart band, or the like.

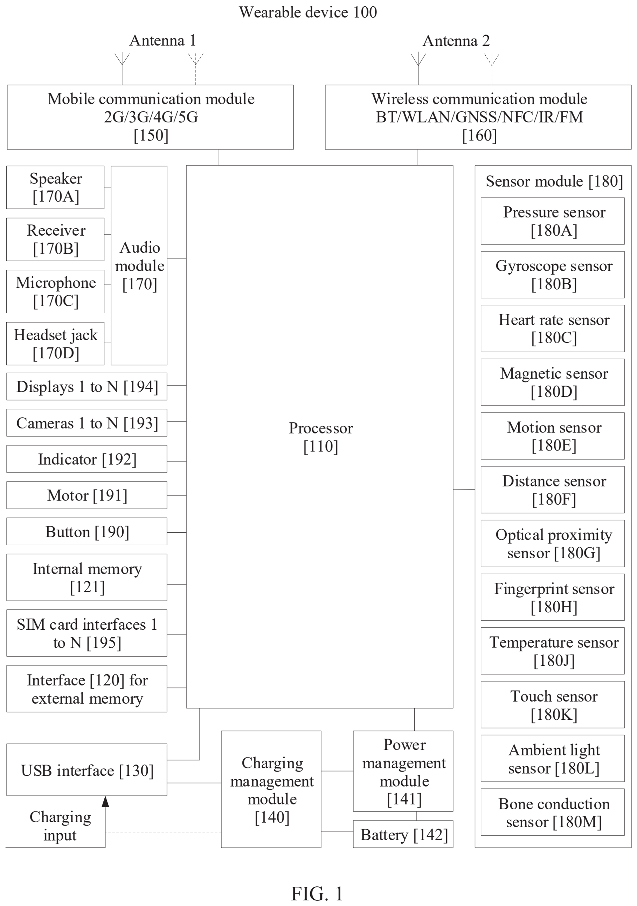

FIG. 1 is merely a diagram of a hardware structure of a wearable device 100 according to an embodiment of this application. Based on the hardware structure shown in FIG. 1, another structural modification may further exist. As shown in FIG. 1, the wearable device 100 may include a processor 110, an interface 120 for external memory, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identity module (subscriber identity module, SIM) card interface 195, and the like. The sensor module 180 may include one or more of the following: a pressure sensor 180A, a gyroscope sensor 180B, a heart rate sensor 180C, a magnetic sensor 180D, a motion sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

The following describes in detail the components of the wearable device 100 shown in FIG. 1.

The processor 110 may include one or more processing units. For example, the processor 110 may include an application processor (application processor, AP), a modem processor, a graphics processing unit (graphics processing unit, GPU), an image signal processor (image signal processor, ISP), a controller, a memory, a video codec, a digital signal processor (digital signal processor, DSP), a baseband processor, a neural-network processing unit (neural-network processing unit, NPU), and/or the like. Different processing units may be independent devices, or may be integrated into one or more processors. In some embodiments, the wearable device 100 may alternatively include one or more processors 110. The processor may be a nerve center and a command center of the wearable device 100. The processor may generate an operation control signal based on instruction operation code and a time sequence signal, to complete control of instruction fetching and instruction execution.

A memory may be further disposed in the processor 110, and is configured to store instructions and data. In some embodiments, the memory in the processor 110 is a cache. The memory may store instructions or data just used or cyclically used by the processor 110. If the processor 110 needs to use the instructions or the data again, the instructions or the data may be directly invoked from the memory, thereby avoiding repeated access, reducing a waiting time of the processor 110, and improving system efficiency.

In some embodiments, the processor 110 may include one or more interfaces. For example, the interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like. It may be understood that an interface connection relationship between the modules shown in this embodiment of this application is merely an example for description, and does not constitute a limitation on the structure of the wearable device 100.

The charging management module 140 is configured to receive a charging input from a charger. The power management module 141 is configured to connect to the battery 142, the charging management module 140, and the processor 110. A wireless communication function of the wearable device 100 may be implemented through the antenna 1, the antenna 2, the mobile communication module 150, the wireless communication module 160, the modem processor, the baseband processor, and the like.

The antenna 1 and the antenna 2 are configured to: transmit and receive an electromagnetic wave signal. Each antenna in the wearable device 100 may be configured to cover one or more communication frequency bands. Different antennas may be further reused, to improve antenna utilization. For example, the antenna 1 may be reused as a diversity antenna of a wireless local area network. In some other embodiments, the antenna may be used in combination with a tuning switch.

The mobile communication module 150 may provide a wireless communication solution that is applied to the wearable device 100 and that includes 2G/3G/4G/5G or the like. The mobile communication module 150 may include at least one filter, a switch, a power amplifier, a low noise amplifier (low noise amplifier, LNA), and the like. The mobile communication module 150 may receive an electromagnetic wave through the antenna 1, perform processing such as filtering or amplification on the received electromagnetic wave, and transmit the electromagnetic wave to the modem processor for demodulation. The mobile communication module 150 may further amplify a signal modulated by the modem processor, and convert the signal into an electromagnetic wave for radiation through the antenna 1. In some embodiments, at least some functional modules of the mobile communication module 150 may be disposed in the processor 110. In some embodiments, at least some functional modules of the mobile communication module 150 may be disposed in a same device as at least some modules of the processor 110.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal. Then, the demodulator transmits the low-frequency baseband signal obtained through demodulation to the baseband processor for processing. The low-frequency baseband signal is processed by the baseband processor and then transmitted to the application processor. The application processor outputs a sound signal by an audio device (which is not limited to the speaker 170A, the receiver 170B, or the like), or displays an image or a video via the display 194. In some embodiments, the modem processor may be an independent device. In some other embodiments, the modem processor may be independent of the processor 110, and is disposed in a same device as the mobile communication module 150 or another functional module.

The wireless communication module 160 may provide a wireless communication solution that is applied to the wearable device 100 and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module 160 may be one or more devices integrating at least one communication processing module. The wireless communication module 160 receives an electromagnetic wave through the antenna 2, performs frequency modulation and filtering processing on an electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation through the antenna 2.

In some embodiments, in the wearable device 100, the antenna 1 is coupled to the mobile communication module 150, and the antenna 2 is coupled to the wireless communication module 160, so that the wearable device 100 can communicate with a network and another device by using a wireless communication technology.

The wearable device 100 implements a display function via the GPU, the display 194, the application processor, and the like. The display 194 is configured to display an image, a video, and the like. The display 194 includes a display panel. The display panel may be a liquid crystal display (liquid crystal display, LCD), an organic light-emitting diode (organic light-emitting diode, OLED), an active-matrix organic light-emitting diode (active-matrix organic light-emitting diode, AMOLED), a flexible light-emitting diode (flexible light-emitting diode, FLED), a mini-LED, a micro-LED, a micro-OLED, a quantum dot light-emitting diode (quantum dot light-emitting diode, QLED), or the like. In some embodiments, the wearable device 100 may include one or N displays 194, where N is a positive integer greater than 1.

In some other embodiments, the wearable device 100 may implement a photographing function via the ISP, the camera 193, the video codec, the GPU, the display 194, the application processor, and the like.

The interface 120 for external memory may be configured to connect to an external memory card, for example, a micro SD card, to extend a storage capability of the wearable device 100. The external memory card communicates with the processor 110 through the interface 120 for external memory, to implement a data storage function. For example, files such as music and videos are stored in the external storage card.

The internal memory 121 may be configured to store computer-executable program code. The executable program code includes instructions. The internal memory 121 may include a program storage area and a data storage area. The program storage area may store an operating system and an application required by at least one function. In addition, the internal memory 121 may include a high-speed random access memory, or may include a nonvolatile memory, for example, at least one magnetic disk storage device, a flash memory, or a universal flash storage (universal flash storage, UFS). The processor 110 runs instructions stored in the internal memory 121 and/or instructions stored in the memory disposed in the processor, to perform various function applications and data processing of the wearable device 100.

The wearable device 100 may implement an audio function, for example, music playing and recording, via the audio module 170, the speaker 170A, the receiver 170B, the microphone 170C, the headset jack 170D, the application processor, and the like.

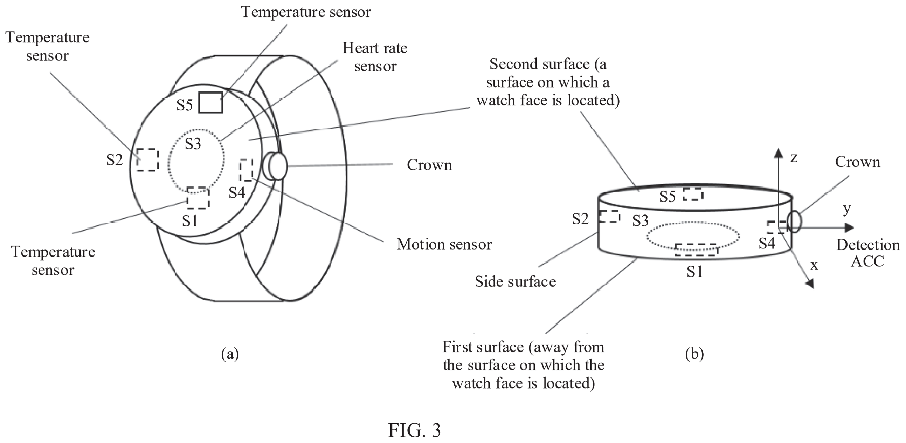

In some embodiments, the wearable device 100 may include one or more heart rate sensors 180C. When the wearable device 100 is worn on a human body, a heart rate of the human body may be measured by using the one or more heart rate sensors 180C, for example, a heart rate sensor S3 included in a wearable device shown in FIG. 3.

In some embodiments, the wearable device 100 may include one or more motion sensors 180E. The wearable device 100 may detect an acceleration parameter of the wearable device 100 by using the motion sensor, to determine a pose of the wearable device 100, for example, a motion sensor S4 included in the wearable device shown in FIG. 3.

In some embodiments, the wearable device 100 may include one or more temperature sensors 180J. When the one or more temperature sensors 180J are in contact with skin of a human body, the wearable device 100 performs body temperature measurement on the human body by using the one or more temperature sensors 180J. When the one or more temperature sensors 180J are not in contact with the human body, for example, in air, the wearable device 100 measures an ambient temperature by using the one or more temperature sensors 180J, for example, a temperature sensor S1, a temperature sensor S1, and a temperature sensor S5 that are included in the wearable device shown in FIG. 3.

The wearable device 100 may receive an input of the button 190, and generate a button signal input related to a user setting and function control of the wearable device 100. For example, the button 190 may include a power button, a volume up button, a volume down button, and the like disposed on a right side of the wearable device.

Although not shown in FIG. 1, the wearable device 100 may further include a Bluetooth apparatus, a positioning apparatus, a flash, a micro projection apparatus, a near field communication (near field communication, NFC) apparatus, and the like. Details are not described herein.

It may be understood that the structure shown in this embodiment of this application does not constitute a specific limitation on the wearable device 100. In some other embodiments of this application, the wearable device 100 may include more or fewer components than those shown in FIG. 1, or some components may be combined, or some components may be split, or there may be a different component layout. The components shown in FIG. 1 may be implemented by hardware, software, or a combination of software and hardware.

The following embodiments may be all implemented by the wearable device 100 having the foregoing hardware structure.

This embodiment of this application is applied to a scenario in which a wearable device is used to perform body temperature measurement on a human body. For example, when the wearable device is worn on the human body, a temperature sensor in the wearable device may be used to perform body temperature measurement on a measured part of the human body. The measured part may include but is not limited to parts such as a wrist, a neck, an ear cavity, an axilla (or armpit), and a forehead.

Scenario 1: When the wearable device is used to perform body temperature measurement on a distal part (for example, a wrist) of the human body or a part (for example, a neck) that is exposed to air and that is prone to a temperature deviation from a core temperature of the human body, a body temperature measurement value obtained through measurement is susceptible to various factors such as an environment change, humidity, and clothing cover, and differs substantially from the core temperature of the human body. In the following, these parts that are prone to a temperature deviation from the core temperature of the human body are collectively referred to as a first measured part. The first measured part includes but is not limited to parts such as a wrist and a neck. In this embodiment of this application, after temperature measurement is performed on the first measured part by using the wearable device to obtain a measured value, the measured value is calibrated, to obtain an accurate body temperature of the human body.

Scenario 2: When the wearable device is used to perform body temperature measurement on some parts that are close to a core temperature of the human body (referred to as a second measured part below), the second measured part includes but is not limited to parts such as an axilla (or armpit) and a forehead. In this embodiment of this application, after temperature measurement is performed on the second measured part by using the wearable device to obtain a measured value, the measured value of the second measured part is used as a body temperature of the human body.

The following provides a body temperature measurement method applicable to the scenario 1.

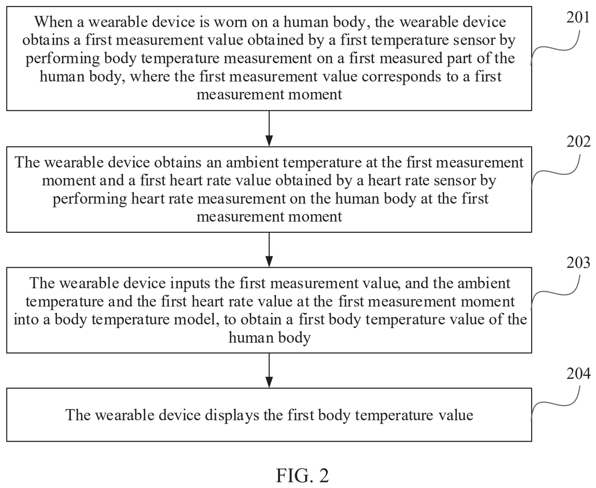

FIG. 2 is a schematic flowchart of a body temperature measurement method according to an embodiment of this application. The method may be performed by a wearable device or a chip or a component in the wearable device. The following embodiments are described by using an example in which the method is performed by the wearable device.

Step 201: When the wearable device is worn on a human body, the wearable device obtains a first measurement value obtained by a first temperature sensor by performing body temperature measurement on a first measured part of the human body, where the first measurement value corresponds to a first measurement moment.

For example, when being worn on the human body, the wearable device may perform temperature measurement on the first measured part of the human body in real time by using the first temperature sensor, or may perform temperature measurement on the first measured part of the human body periodically, to obtain temperature measurement values of the first measured part at different moments. Alternatively, after receiving a measurement instruction, the wearable device may perform temperature measurement on the first measured part by using the first temperature sensor in response to the measurement instruction, to obtain the first measurement value.

With reference to the wearable device shown in FIG. 3, the following describes the first temperature sensor included in the wearable device. In FIG. 3, a smartwatch is used as an example of the wearable device. A form of the wearable device is not limited in this application. (a) in FIG. 3 is a front view of the wearable device, and (b) in FIG. 3 is a side view of the wearable device.

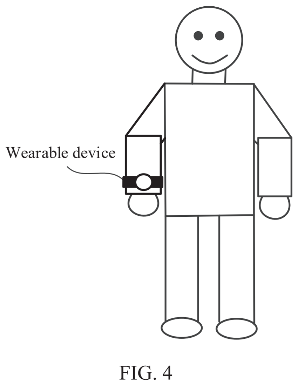

As shown in (a) in FIG. 3, the wearable device may include a temperature sensor S1. As shown in (b) in FIG. 3, the temperature sensor S1 is disposed on a first surface of the wearable device, and the first surface is a surface that is of the wearable device and that is away from a surface on which a watch face (or a display) is located. FIG. 4 is a diagram in which the wearable device shown in FIG. 3 is worn on a wrist of the human body. When the wearable device is worn on the wrist of the human body, the first surface is in contact with skin of the wrist of the human body, and the temperature sensor S1 may measure a wrist temperature of the human body. In this way, when the wearable device is worn on the wrist of the human body, the wrist temperature can be directly measured, and the wearable device does not need to be removed from the wrist to measure a body temperature. In some other embodiments, the wearable device may alternatively be removed from the wrist of the human body, and the temperature sensor S1 located on the first surface of the wearable device is in contact with another part (for example, a forehead or a neck) of the human body, to measure a temperature of the another part of the human body.

In some embodiments, as shown in (a) in FIG. 3, the wearable device may further include a temperature sensor S2, which is disposed at any position on a side surface of the wearable device. For example, as shown in (b) in FIG. 3, the temperature sensor S2 is located at a position that is on the side surface and that is opposite to a watch crown. When the wearable device is worn on the wrist of the human body, the first surface is in contact with skin of the wrist of the human body, and the temperature sensor S2 located on the side surface of the wearable device is generally not in contact with skin of the human body, but is in an air environment, and may measure an ambient temperature of an environment in which the human body is located. In some other embodiments, the wearable device may alternatively be removed from the wrist of the human body, so that the temperature sensor S2 located on the side surface is in contact with skin of another part of the human body, to measure a body temperature of the another part of the human body. For example, the temperature sensor S2 in the wearable device is in contact with the first measured part, to measure a temperature of the first measured part.

In some embodiments, as shown in (a) in FIG. 3, the wearable device may further include a temperature sensor S5. As shown in (b) in FIG. 3, the temperature sensor S5 is disposed on a second surface of the wearable device, and the second surface is the surface on which the watch face (or the display) of the wearable device is located. When the wearable device is worn on the wrist of the human body, the first surface is in contact with the skin of the wrist of the human body, and the second surface is not in contact with the skin of the wrist of the human body. Therefore, the temperature sensor S5 is in the air environment, and may measure the ambient temperature of the environment in which the human body is located. In some other embodiments, the wearable device may alternatively be removed from the wrist of the human body, and the second surface of the wearable device is in contact with the first measured part of the human body, so that the temperature sensor S5 may measure a temperature of the first measured part of the human body.

It should be understood that the temperature sensor S1 may alternatively be disposed at a position that is inside the wearable device and that is close to the first surface, and the temperature sensor S1 is not visible to a user from an exterior of the wearable device. When the wearable device is worn on the wrist of the human body, the temperature sensor S1 is not directly in contact with the skin of the wrist, but a skin temperature is transferred to the temperature sensor S1 via the first surface of the wearable device. Similarly, the temperature sensor S2 may alternatively be disposed at any position that is inside the wearable device and that is close to the side surface, and the temperature sensor S5 may alternatively be disposed at a position that is inside the wearable device and that is close to the second surface. The temperature sensor S5 is not visible to the user from the exterior of the wearable device. When the second surface of the wearable device is in contact with skin of the human body, a skin temperature is transferred from the second surface to the temperature sensor S5.

With reference to FIG. 3, that the wearable device in step 201 is the wearable device shown in FIG. 3 is used as an example. The first temperature sensor in step 201 may be any one of the temperature sensor S1, the temperature sensor S2, and the temperature sensor S5 in FIG. 3. The user may select, based on a position of the first measured part on the human body, one temperature sensor from the temperature sensor S1, the temperature sensor S2, and the temperature sensor S5, to perform temperature measurement on the first measured part.

Step 202: The wearable device obtains an ambient temperature at the first measurement moment and a first heart rate value obtained by a heart rate sensor by performing heart rate measurement on the human body at the first measurement moment.

Herein, the ambient temperature at the first measurement moment may be an actual temperature of the environment in which the human body is located at the first measurement moment, or may be a preset fixed temperature value. The preset fixed temperature value may be a default value, or may be set by the user.

The following describes a possible implementation of obtaining the ambient temperature at the first measurement moment by using an example in which the ambient temperature at the first measurement moment in step 202 is the actual temperature of the environment in which the human body is located at the first measurement moment.

In a possible implementation, the wearable device may obtain the ambient temperature at the first measurement moment from another device (for example, a mobile terminal). For example, the mobile terminal periodically sends the ambient temperature to the wearable device. For another example, the wearable device sends ambient temperature query information to the mobile terminal when performing body temperature measurement at the first measurement moment. The temperature query information is used to query the ambient temperature at the first measurement moment, so that the wearable device obtains the ambient temperature at the first measurement moment from the mobile terminal.

In another possible implementation, the wearable device may further include a second temperature sensor, and the wearable device may obtain the ambient temperature at the first measurement moment through measurement by using the second temperature sensor. For example, the second temperature sensor is the temperature sensor S2 shown in FIG. 3. In this way, compared with obtaining the ambient temperature at the first measurement moment from the another device, in this implementation, the wearable device may measure the ambient temperature at the first measurement moment by using the second temperature sensor, thereby reducing a quantity of interactions with the another device.

In some embodiments, the wearable device may further include the heart rate sensor, for example, a heart rate sensor S3 shown in FIG. 3. As shown in FIG. 3, the heart rate sensor S3 is disposed on the first surface of the wearable device, that is, the surface that is of the wearable device and that is away from the watch face. For example, when the wearable device is worn on the wrist of the human body, the heart rate sensor S3 is in contact with the skin of the wrist, and may monitor a heart rate of the human body. In some embodiments, the heart rate sensor S3 may obtain a heart rate value that is of the human body and that is measured in real time, and then store the heart rate value in a storage apparatus (for example, a storage or a memory) of the wearable device. It should be understood that the heart rate sensor S3 may alternatively be disposed at a position that is inside the wearable device and that is close to the first surface, and the heart rate sensor S3 is not visible to the user from the exterior of the wearable device.

Step 203: The wearable device inputs the first measurement value, and the ambient temperature and the first heart rate value at the first measurement moment into a body temperature model, to obtain a first body temperature value of the human body.

The body temperature model may have the following two possible implementations.

In a possible implementation 1, a structure parameter of the body temperature model is a preset value. In the following, the body temperature model with the structure parameter being the preset value is referred to as a preset body temperature model, and the preset body temperature model may be preconfigured in the wearable device.

For example, the preset body temperature model may be represented by the following Formula (1): ${T}_{b} = α \times f \left({T}_{s}, {T}_{e}\right) + β \times g \left({H}_{r}\right)$

In the Formula (1), a body temperature *T_{b}* includes two parts: A first part is a submodel *α* × *f*(*Tₛ, Tₑ*)*,* represented by a wrist temperature *Tₛ* and an ambient temperature *Tₑ,* and a second part is a submodel *β* × *g*(*Hᵣ*) represented by a heart rate *Hᵣ,* where α and β are preset coefficients, and the parameter β represents a reciprocal of a slope of an increase in heart rate caused by a rise in body temperature.

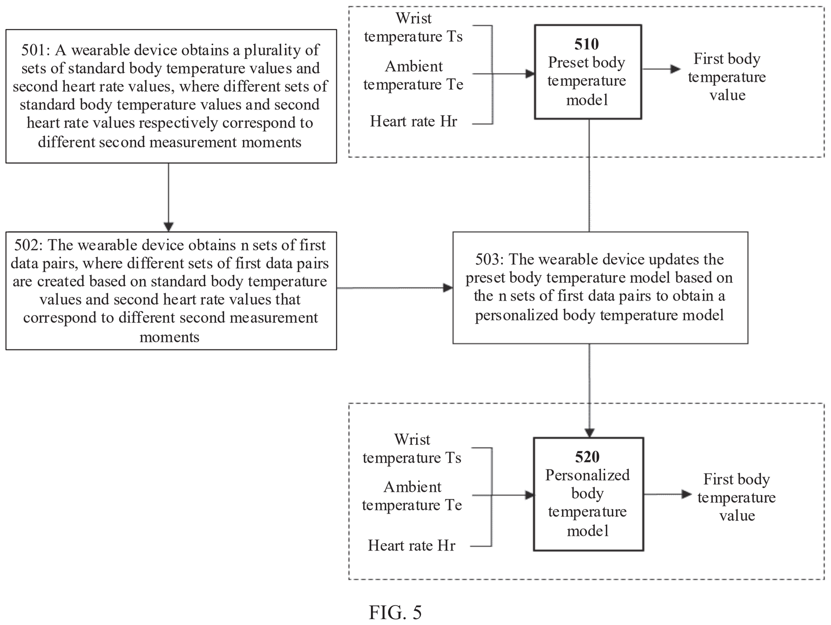

In a preset body temperature model 510 shown in FIG. 5, after the wearable device obtains the first measurement value at the first measurement moment, the ambient temperature at the first measurement moment, and the first heart rate value at the first measurement moment, the first measurement value obtained by the wearable device, the ambient temperature obtained by the wearable device, and the first heart rate value obtained by the wearable device are respectively used as the wrist temperature *Tₛ*, the ambient temperature *Tₑ,* and the heart rate *Hᵣ*, and are input to the preset body temperature model 510, to predict the first body temperature value of the human body at the first measurement moment.

In a possible implementation 2, a structure parameter of the body temperature model is generated by performing model training based on standard body temperature values and heart rate values of the human body in historical duration. The body temperature model is referred to as a personalized body temperature model below. The personalized body temperature model may be obtained by updating a structure parameter of a preset body temperature model based on the standard body temperature value and the heart rate value of the human body in the historical duration.

The following describes a process of creating the personalized body temperature model with reference to FIG. 5. It should be understood that the process of creating the personalized body temperature model may be triggered by the user, for example, triggered in response to a user instruction. Alternatively, the process of creating the personalized body temperature model may be automatically triggered by the wearable device, for example, triggered when a quantity of data pairs including a plurality of sets of standard body temperature values and heart rate values of the human body reaches a specific quantity threshold.

As shown in FIG. 5, the process of creating the personalized body temperature model includes the following steps.

Step 501: The wearable device obtains a plurality of sets of standard body temperature values and second heart rate values, where different sets of standard body temperature values and second heart rate values respectively correspond to different second measurement moments, and a standard body temperature value and a second heart rate value in each set correspond to a same second measurement moment.

The standard body temperature value in this application may be a temperature of a part that has a temperature close to a core temperature in the human body and on which body temperature measurement can be performed by using a temperature measurement apparatus. Preset parts may include but are not limited to at least one of the following: an axilla (or armpit), an oral cavity, an ear cavity, a forehead, and a rectum. Temperatures of these preset parts are close to the core temperature of the human body. Therefore, the temperatures of the preset parts are used as standard body temperature values to create the personalized body temperature model, so that the personalized body temperature model that can accurately predict the body temperature of the human body can be obtained. Standard body temperature values corresponding to different parts may be measured by using different temperature measurement apparatuses. For example, the temperature of the axilla (or armpit) may be measured by using a mercury thermometer, a wearable device, or the like, the temperature of the oral cavity may be measured by using a mercury thermometer, the temperature of the ear cavity may be measured by using an ear thermometer, the temperature of the forehead may be measured by using a forehead thermometer, a wearable device, or the like, and the temperature of the rectum may be measured by using a mercury thermometer.

In this embodiment of this application, the wearable device may obtain the standard body temperature value in a plurality of manners.

In a possible implementation, the wearable device receives a standard body temperature value entered by the user. The entered standard body temperature value may be obtained by the user by performing body temperature measurement on the preset part of the human body by using the mercury thermometer, the ear thermometer, the forehead thermometer, or another body temperature measurement tool. When entering the standard body temperature value, the user may further enter a second measurement moment corresponding to each standard body temperature value, and then the wearable device may obtain heart rate values obtained by a heart rate sensor through measurement at different second measurement moments.

In another possible implementation, the user may place the wearable device on the preset part to obtain the standard body temperature value through measurement. The wearable device may record the obtained standard body temperature value through measurement and a corresponding second measurement moment, and obtain, from the background, a heart rate value obtained by the heart rate sensor through measurement at the second measurement moment.

Step 502: The wearable device obtains n sets of first data pairs, where different sets of first data pairs are created based on the standard body temperature values and the second heart rate values that correspond to different second measurement moments.

The first data pair may also be referred to as a body temperature-heart rate pair, and may be represented as (*Tₙ,* H*ᵣₙ*), where n is a positive integer. When the wearable device obtains a sufficient quantity of body temperature-heart rate pairs, for example, n body temperature-heart rate pairs, which are respectively (*T*₁, H_{*r*1}), (*T*₂, H_{*r*2}), ..., and (*Tₙ,* H*ᵣₙ*), the wearable device may automatically create the personalized body temperature model, or the wearable device may prompt the user to create the personalized body temperature model, and then the user triggers creation of the personalized body temperature model. In an implementation, the wearable device may display second prompt information, where the second prompt information is used to prompt to update the structure parameter of the body temperature model. Then, the wearable device receives a first instruction, where the first instruction instructs the wearable device to update the body temperature model. In response to the first instruction, the wearable device updates the structure parameter of the body temperature model based on at least one first data pair. In some other embodiments, the wearable device may alternatively not prompt the user to create the personalized body temperature model, and creation of the personalized body temperature model is triggered by the user. A specific manner of triggering creation of the personalized body temperature model is not limited in this application.

Step 503: The wearable device updates the preset body temperature model based on the n sets of first data pairs to obtain the personalized body temperature model.

In a possible implementation, the wearable device may perform pairwise calculation of a coefficient *βᵢ₋ⱼ* on the n sets of first data pairs, and then take the average.

For example, a personalized coefficient *β*₁₋₂ is calculated based on a first data pair (*T*₁, H_{*r*1}) and a first data pair (*T*₂, H_{*r*2}) by using the following Formula (2): ${β}_{1 - 2} = \frac{{T}_{2} - {T}_{1}}{{H}_{r 2} - {H}_{r 1}}$

Pairwise calculation of *βᵢ₋ⱼ* is performed on the foregoing n first data pairs (*T*₁, H_{*r*1}), (*T*₂, H_{*r*2}), ..., and (*Tₙ,* H*ᵣₙ*), to obtain *c = n* × (*n* - 1)/2 pieces of *βᵢ₋ⱼ* values, which are respectively *β*₁₋₂, *β*₁₋₃, ..., *β*_{1-*n*}, *β*₂₋₃, *β*₂₋₄, ..., *β*₂*₋ₙ,* ..., and *β*₍ₙ₋₁₎₋ₙ. Then, the c *βᵢ₋ⱼ* values are averaged to obtain a final personalized parameter *βₚ,* which is represented by the following Formula (3): ${β}_{p} = \frac{1}{c} \sum {β}_{i - j}$

After obtaining the personalized parameter *βₚ*, the wearable device updates the parameter β in the preset body temperature model to the personalized parameter *βₚ*, to obtain the personalized body temperature model, which may be represented by the following Formula (4): ${T}_{b} = α \times f \left({T}_{s}, {T}_{e}\right) + {β}_{p} \times g \left({H}_{r}\right)$

In some other embodiments, the personalized parameter *βₚ* may alternatively be obtained by fitting based on the n sets of first data pairs through a least square method.

According to the personalized body temperature model 520 shown in FIG. 5, after the wearable device obtains the first measurement value, the ambient temperature at the first measurement moment, and the first heart rate value at the first measurement moment, the first measurement value obtained by the wearable device, the ambient temperature obtained by the wearable device, and the first heart rate value obtained by the wearable device are respectively used as a wrist temperature *Tₛ*, an ambient temperature *Tₑ,* and a heart rate *Hᵣ*, and are input to the personalized body temperature model 520, to obtain the first body temperature value.

After the first body temperature value of the human body at the first measurement moment is predicted by using the preset body temperature model 510 or the personalized body temperature model 520, step 204 may be performed.

Step 204: The wearable device displays the first body temperature value.

In a possible implementation, before step 203, the wearable device may further determine whether the personalized body temperature model has been created. The following describes two cases.

Case 1: If the wearable device has created the personalized body temperature model, the wearable device inputs the first measurement value, and the ambient temperature and the first heart rate value at the first measurement moment into the personalized body temperature model, to obtain the first body temperature value of the human body, and displays the first body temperature value. Because the structure parameter of the personalized body temperature model is generated by performing an operation based on the standard body temperature values and the heart rate values of the human body in the historical duration, the first body temperature value of the human body that is predicted by using the personalized body temperature model is relatively close to the core temperature of the human body.

Case 2: If the wearable device has not created the personalized body temperature model, the wearable device inputs the first measurement value, and the ambient temperature and the first heart rate value at the first measurement moment into the preset body temperature model, to obtain the first body temperature value of the human body.

When the wearable device is worn on the first measured part of the human body, because a body temperature measurement value obtained through measurement of the first measured part differs substantially from the core temperature of the human body, to obtain a more accurate body temperature of the human body, the wearable device may display first prompt information after the first body temperature value is predicted by using the preset body temperature model. The first prompt information is used to prompt the user to enter a standard body temperature value, for example, prompt the user to perform body temperature measurement on a preset part and enter a standard body temperature value, for another example, prompt the user to perform body temperature measurement by using a preset measurement apparatus and enter a standard body temperature value, and for still another example, prompt the user to perform body temperature measurement on a preset part by using a preset measurement apparatus and enter a standard body temperature value. A preset measurement method may be that a body temperature measurement tool like a mercury thermometer, an ear thermometer, or a forehead thermometer is used to perform body temperature measurement on an axilla (or armpit) part, an oral cavity part, an ear cavity part, a forehead part, a rectum part, or the like of the human body. Optionally, the wearable device may alternatively be placed on an axilla (or armpit) part or a forehead part to measure a body temperature, to directly obtain a standard body temperature value. After obtaining the standard body temperature value, the wearable device may further display the standard body temperature value. These accurate body temperature values may be used as standard temperature values, to create the personalized body temperature model together with heart rate values obtained through measurement at corresponding measurement moments.

In the foregoing embodiment, an example in which the wearable device prompts the user to enter an accurate body temperature value after obtaining the first body temperature value by using the preset body temperature model is used for description. In some other embodiments, after obtaining the first body temperature value through prediction by using the preset body temperature model, the wearable device may display the first prompt information when determining that the first body temperature value is greater than or equal to a first threshold, where the first prompt information is used to prompt the user to enter a standard body temperature value. When it is determined that the first body temperature value is less than the first threshold, the first prompt information is not displayed, that is, the user is not prompted to enter a standard body temperature value.

The first threshold may be a default value, or may be set by the user. For example, the first threshold is set to 36.5°C. The wearable device measures the first measurement value after being worn on the human body, and then obtains the first body temperature value through prediction by using the preset body temperature model. If the predicted first body temperature value is greater than 36.5°C, the user is prompted to enter a standard body temperature value. For another example, the first threshold is set to a larger value, for example, set to 37.2°C. In this way, when the first body temperature value predicted by the wearable device by using the preset body temperature model is greater than or equal to 37.2°C, it indicates that the human body is in a fever state, and the user is prompted to enter a standard body temperature value. In this case, the entered standard body temperature value is mostly a body temperature value of the human body in the fever state. For still another example, the first threshold is set to a larger value, for example, set to 38.5°C. In this way, when the first body temperature value predicted by the wearable device by using the preset body temperature model is greater than or equal to 38.5°C, it indicates that the human body is in a high fever state, and the user is prompted to enter a standard body temperature value. In this case, the entered standard body temperature value is mostly a body temperature value of the human body in the high fever state. In specific implementation, the first threshold may be set based on an actual requirement.

Taking an example in which a wearable device is worn on a wrist of a human body below, this application provides a specific example of implementing a body temperature measurement method.

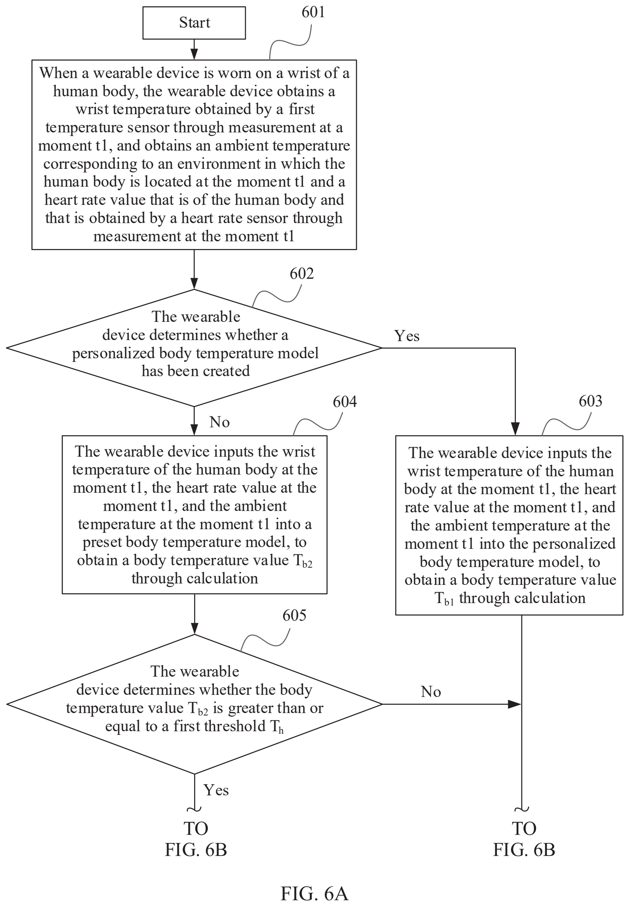

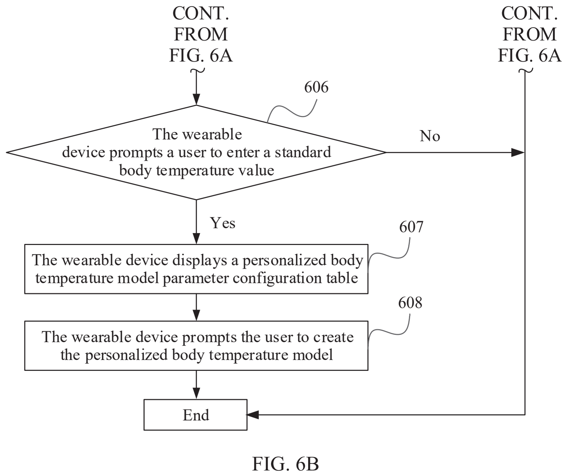

As shown in FIG. 6A and FIG. 6B, the body temperature measurement method includes the following steps.

Step 601: When the wearable device is worn on the wrist of the human body, the wearable device obtains a wrist temperature obtained by a first temperature sensor through measurement at a moment t1, and obtains an ambient temperature of an environment in which the human body is located at the moment t1 and a heart rate value that is of the human body and that is obtained by a heart rate sensor through measurement at the moment t1.

Step 602: The wearable device determines whether a personalized body temperature model has been created; and if the personalized body temperature model has been created, performs step 603; or if the personalized body temperature model has not been created, performs step 604.

Step 603: The wearable device inputs the wrist temperature of the human body at the moment t1, the heart rate value at the moment t1, and the ambient temperature at the moment t1 into the personalized body temperature model, to obtain a body temperature value *T*_{*b*1} through calculation.

Optionally, after the body temperature value *T*_{*b*1} is obtained through calculation in step 603, the wearable device may further present the body temperature value *T*_{*b*1} to a user, and then end the procedure.

Step 604: The wearable device inputs the wrist temperature of the human body at the moment t1, the heart rate value at the moment t1, and the ambient temperature at the moment t1 into a preset body temperature model, to obtain a body temperature value *T*_{*b*2} through calculation.

Step 605: The wearable device determines whether the body temperature value *T*_{*b*2} is greater than or equal to a first threshold *Tₕ*; and if the body temperature value is greater than or equal to the first threshold, performs step 606; or if the body temperature value is less than the first threshold, ends the procedure.

Optionally, if the wearable device determines that the body temperature value *T*_{*b*2} is less than the first threshold *Tₕ*, the wearable device may present the body temperature value *T*_{*b*2} to a user, and then end the procedure.

Step 606: The wearable device prompts the user to enter a standard body temperature value.

Herein, for specific implementation of the standard body temperature value, refer to the foregoing related content. Details are not described herein again.

Step 607: The wearable device displays a personalized body temperature model parameter configuration table.

The personalized body temperature model parameter configuration table may include content such as a measurement position, a measurement moment, a standard body temperature value, and a heart rate value. After the user enters the standard body temperature value and a corresponding second measurement moment, the wearable device may obtain a second heart rate value corresponding to the second measurement moment, form a body temperature-heart rate pair, and present the body temperature-heart rate pair to the user via a display.

The wearable device continuously repeats a process of step 601 to step 607 until a sufficient data volume of body temperature-heart rate pairs is accumulated. After accumulating enough body temperature-heart rate pairs, the wearable device performs step 608.

In a possible implementation, after the personalized body temperature model parameter configuration table is fully populated, step 608 is performed.

In another possible implementation, the wearable device may alternatively perform step 608 when a proportion of a data volume of populated content in the personalized body temperature model parameter configuration table to a capacity of the parameter configuration table is greater than a threshold.

Step 608: The wearable device prompts the user to create the personalized body temperature model, and then ends the procedure.

After step 608, the user may actively trigger creation of the personalized body temperature model, and then the procedure ends.

In some other embodiments, the wearable device may alternatively automatically create the personalized body temperature model when the personalized body temperature model parameter configuration table is fully populated or a proportion of a data volume of populated content to a capacity of the parameter configuration table is greater than a threshold. That is, step 608 is not performed. In this way, the personalized body temperature model can be created without awareness of the user.

In still some other embodiments, the wearable device may alternatively perform step 608 when the personalized body temperature model parameter configuration table is fully populated or a proportion of a data volume of populated content to a capacity of the parameter configuration table is greater than a threshold, and automatically create the personalized body temperature model.

It should be understood that the wearable device may monitor the wrist temperature in real time, or may periodically monitor the wrist temperature. This is not limited in this application.

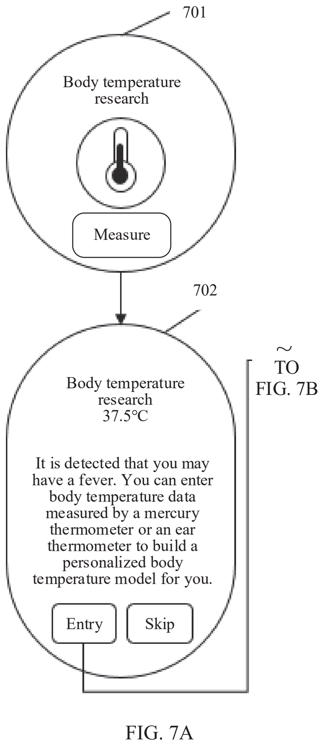

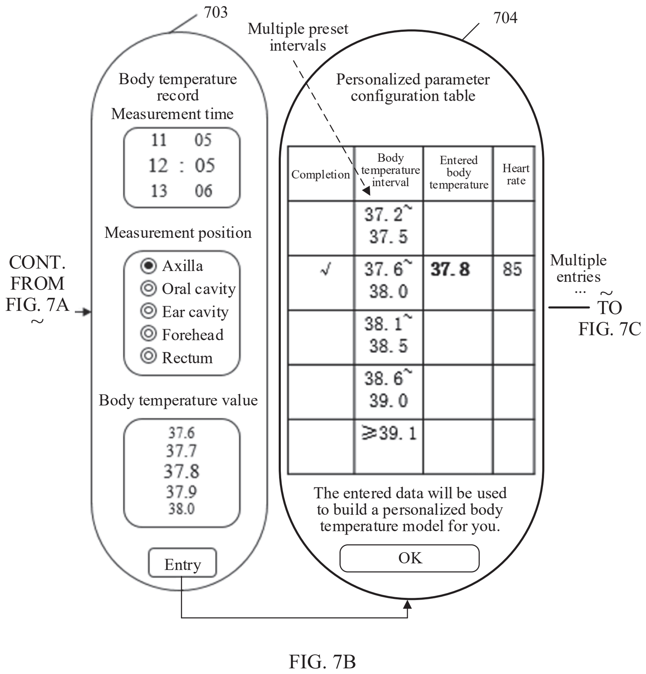

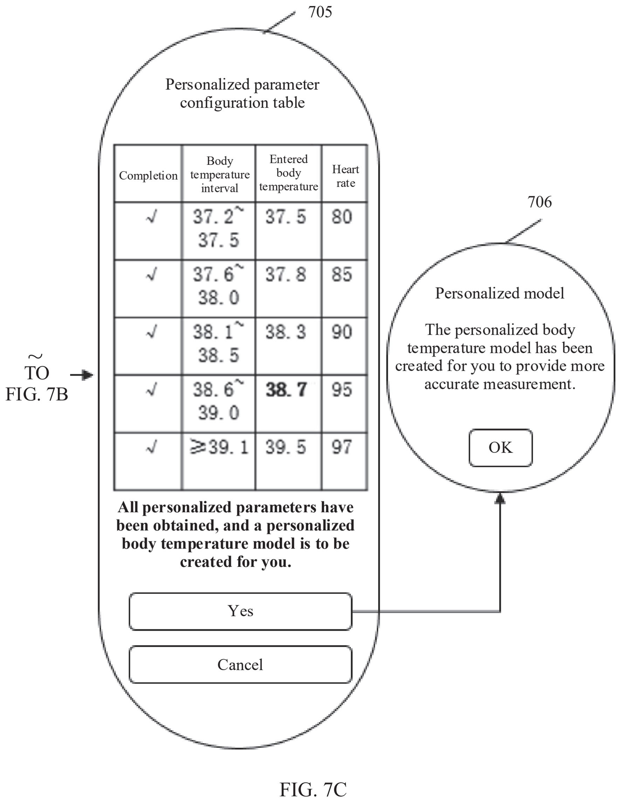

The following describes a process of entering a body temperature record with reference to an example in FIG. 7A, FIG. 7B, and FIG. 7C.

For example, the first threshold is 37.2°C. In a user interface 701 shown in FIG. 7A, when the user uses a body temperature measurement function, and the wearable device has not created the personalized body temperature model for the user, the wearable device may measure a body temperature by using the preset body temperature model, then predict a first body temperature value, and display a user interface 702 shown in FIG. 7A. The user interface 702 includes a body temperature prediction result, namely, 37.5°C, of the preset body temperature model, where the body temperature prediction result (37.5°C) is greater than the first threshold (37.2°C). Optionally, the user interface 702 further includes the following prompt information: "It is detected that you may have a fever. You can enter body temperature data measured by a mercury thermometer or an ear thermometer to build a personalized body temperature model for you". The prompt information in the user interface 702 may alternatively be other content used to prompt to enter a standard body temperature value. This is not limited herein.

The user interface 702 may further include an entry control, and the entry control is configured to trigger entry of a standard body temperature value. For example, the user measures a body temperature by using a mercury thermometer, and may perform an operation on entry space, for example, a tap operation. The wearable device enters a body temperature recording interface 703 in response to the tap operation of the user on the first control. The body temperature recording interface 703 may include measurement time (for example, 11:05, 12:05, or 13:06), a measurement position (for example, an axilla, an oral cavity, an ear cavity, a forehead, or a rectum), a body temperature value (for example, 37.6°C, 37.7°C, 37.8°C, 37.9°C or 38°C), and the like. The user selects a set of body temperature records on the wearable device. For example, the measurement time is 12:05, the measurement position is the axilla, and the body temperature value is 37.8°C. The body temperature recording interface 703 further includes an entry control. The user may perform an operation on entry space in the body temperature recording interface 703, for example, a tap operation. The wearable device displays a user interface 704 shown in FIG. 7B in response to the tap operation of the user on the first control. Each row in the personalized parameter configuration table includes a set of parameters, that is, parameters such as degree of completion, body temperature range, entered body temperature, and heart rate. After the body temperature record is entered, the wearable device may find, in the background based on the measurement time (12:05) entered by the user, a heart rate (for example, 85 bpm) corresponding to the measurement time (12:05). Therefore, a body temperature-heart rate pair (37.8°C-85 bpm) is created for the user, and the result is presented to the user.

The user repeatedly performs the process of recording a body temperature record, and obtains a plurality of body temperature-heart rate pairs. For example, the user accumulates a body temperature-heart rate pair in each of the following five intervals: 37.2°C to 37.5°C, 37.6°C to 38.0°C, 38.1°C to 38.5°C, 38.6°C to 39.0°C, and greater than or equal to 39.1°C, which indicates that sufficient data has been obtained for the user to describe a relationship between a body temperature and a heart rate of the user. The wearable device displays a user interface 705 shown in FIG. 7C. The personalized parameter configuration table in the user interface 705 is fully populated, and the user interface 705 further displays the following prompt information: "All personalized parameters have been obtained, and a personalized body temperature model is to be created for you". The user interface 705 further includes a "yes" control and a "cancel" control that correspond to the prompt information. The "yes" control is configured to trigger creation of the personalized body temperature model. When the user performs an operation on the "yes" control, for example, a tap operation, the wearable device creates the personalized body temperature model in response to the tap operation, and displays a user interface 706 after completing the creation of the personalized body temperature model. The user interface 706 includes prompt information that the creation of the personalized model is completed, for example, "The personalized temperature model has been created for you to provide more accurate measurement".

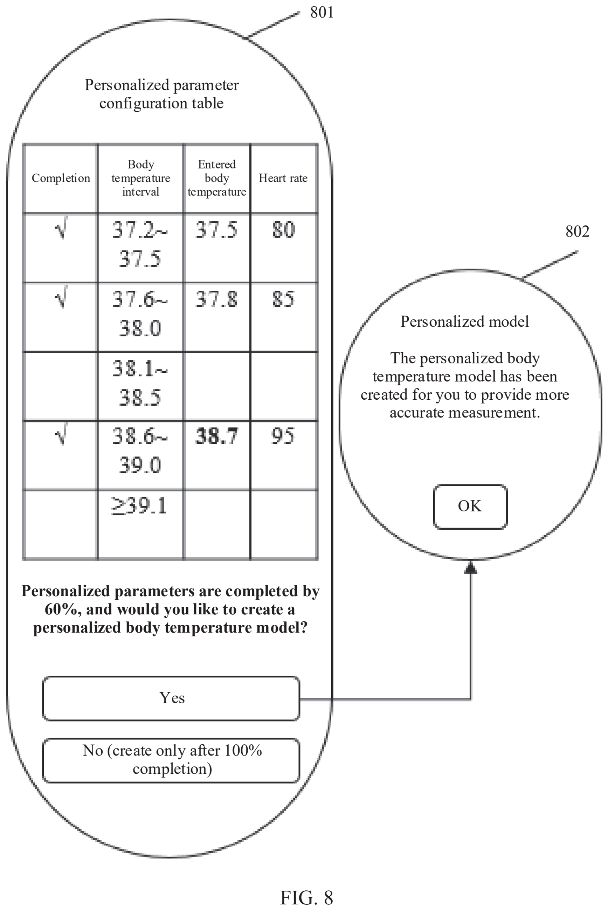

In some other embodiments, when a data volume of entered body temperature-heart rate pairs of the wearable device reaches a specific threshold, the wearable device prompts the user to create the personalized body temperature model. For example, in FIG. 8, after the user has entered three pieces of data, the wearable device prompts the user whether to create a model based on available 60% of data. In a user interface 801 shown in FIG. 8, the wearable device displays the following prompt information: "Personalized parameters are completed by 60%, and would you like to create a personalized body temperature model". The user interface 801 further includes a "yes" control and a "cancel" control that correspond to the prompt information. The "yes" control is configured to trigger creation of the personalized body temperature model. When the user performs an operation on the "yes" control, for example, a tap operation, the wearable device creates the personalized body temperature model in response to the tap operation, and displays a user interface 802 after completing the creation of the personalized body temperature model. For the user interface 802, refer to the related descriptions of the user interface 706 in FIG. 7C. Details are not described herein again. Accuracy of the personalized body temperature model created by using the example shown in FIG. 8 is lower than accuracy of the personalized body temperature model created in FIG. 7A, FIG. 7B, and FIG. 7C. However, the personalized body temperature model can be created more quickly by using the example shown in FIG. 8. In this way, the user can experience the personalized body temperature model more quickly.

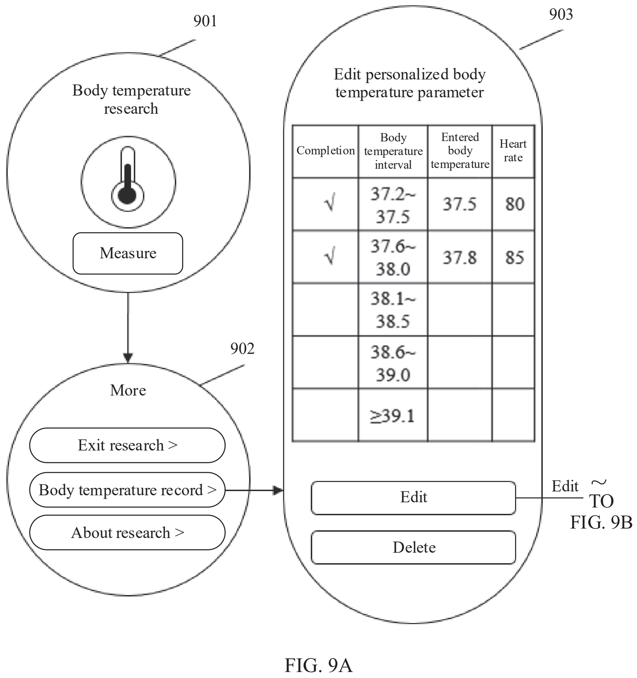

The wearable device displays a user interface 901 shown in FIG. 9A. The user may use the body temperature measurement function. For example, the user may perform an operation on a measurement control in the user interface 901. The wearable device measures a body temperature by using the preset body temperature model in response to the operation on the measurement control. In some other embodiments, when the wearable device does not prompt entry of a standard body temperature value, the user may alternatively actively enter a standard body temperature value. The user may perform an operation on the user interface 901. The wearable device enters, in response to the operation, a user interface 902 shown in FIG. 9A. The user interface 902 includes a body temperature record control. The user may perform an operation on the body temperature record control, and the wearable device displays a user interface 903. The user interface 903 includes a personalized body temperature parameter table. The user may manually edit or delete data of a body temperature-heart rate pair in the personalized body temperature parameter table. The user interface 903 may further include an edit control. The user may perform an operation on the edit control, for example, a tap operation. The wearable device enters, in response to the tap operation, a mode in which the personalized body temperature parameter table is editable. For example, the user enters a body temperature-heart rate pair (38.2°C-88 bpm) in the personalized body temperature parameter table, and the wearable device displays a user interface 904. The user interface 904 may further include a refresh personalized model control. When the wearable device has created the personalized body temperature model, a body temperature-heart rate pair is added. The user may perform an operation on the refresh personalized model control, to update a parameter of the created personalized body temperature model, to obtain a personalized body temperature model with higher accuracy.

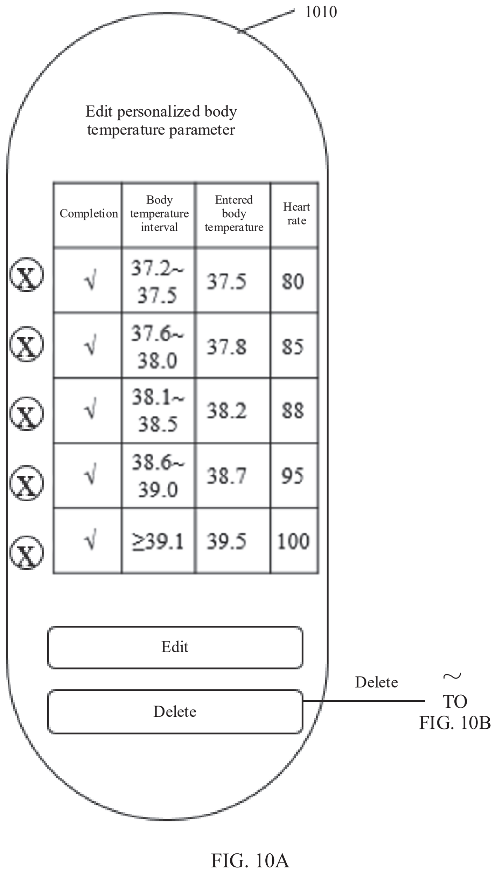

When the wearable device displays the personalized body temperature parameter table, the user may further delete data in the personalized body temperature parameter table. For example, the wearable device displays a user interface 1010 shown in FIG. 10A. The user interface 1010 may include a delete control. When the user selects a body temperature-heart rate pair (for example, 38.7°C-95 bpm) in the personalized body temperature parameter table, the user may perform an operation on the delete control, for example, a tap operation, and the wearable device deletes the body temperature-heart rate pair (for example, 38.7°C-95 bpm) in response to the tap operation, and displays a user interface 1020. The user interface 1020 may further include a refresh personalized model control. When the wearable device has created the personalized body temperature model, a body temperature-heart rate pair is deleted. The user may also perform an operation on the refresh personalized model control, to update a parameter of the created personalized body temperature model.

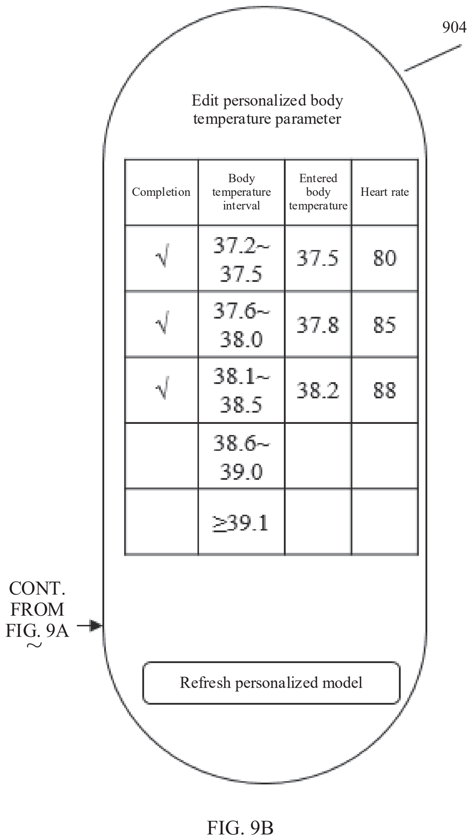

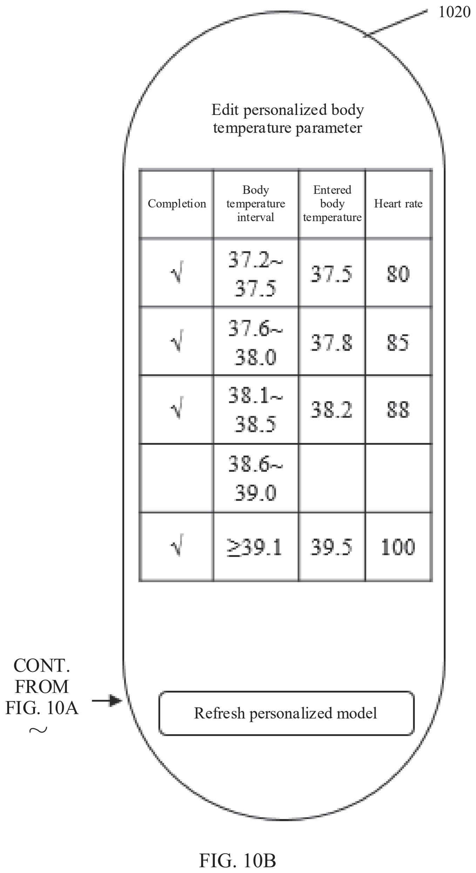

According to the examples shown in FIG. 9A and FIG. 9B, and FIG. 10A and FIG. 10B, the user may actively enter a page of the personalized body temperature parameter table, to quickly complete updating of a personalized body temperature parameter. After adding or deleting a record, the user may alternatively refresh the personalized body temperature model.

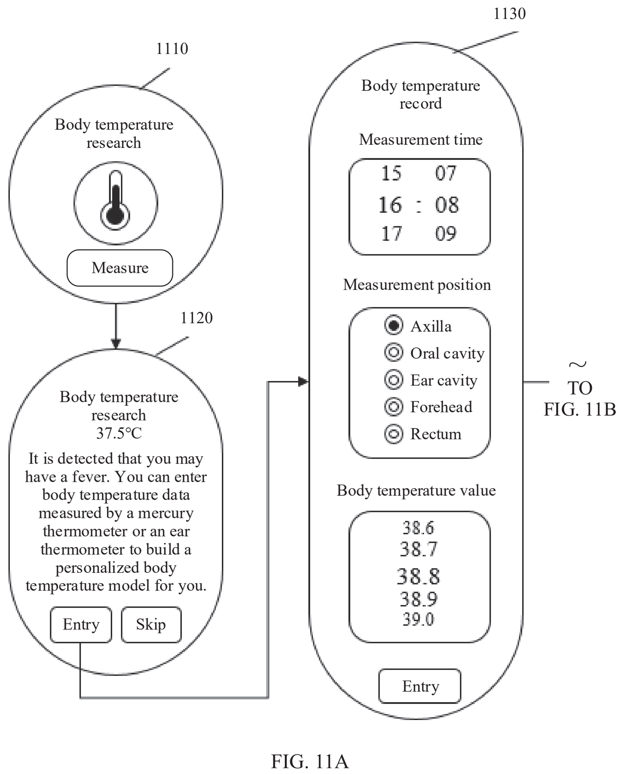

In some other embodiments, when the first body temperature value obtained through measurement is greater than or equal to a second threshold, the wearable device may further prompt the user to enable a temperature measurement reminder function. The temperature measurement reminder function may periodically remind the user to measure a body temperature. A name of the temperature measurement reminder function is not limited in this application, for example, may also be referred to as a scheduled reminder function for temperature measurement, or may also be referred to as a scheduled temperature measurement reminder function. For example, the second threshold is set to a temperature value in a high fever state, for example, 38.5°C, indicating the high fever state. The wearable device displays a user interface 1110 shown in FIG. 11A. The user may use the body temperature measurement function. For example, the user may perform an operation on a measurement control in the user interface 1110. The wearable device measures a body temperature by using the preset body temperature model in response to the operation on the measurement control. When the wearable device detects that the body temperature of the user is high, for example, detects that the body temperature of the user is 38.5°C in a user interface 1120 shown in FIG. 11A, the user enters a body temperature of 38.8°C in a user interface 1130, indicating that the user is in a high fever state. After the body temperature is entered in the user interface 1130, the wearable device displays a user interface 1140. The user interface 1140 includes a personalized parameter data configuration table and the following prompt information used to prompt to enable the temperature measurement reminder function: "You are currently experiencing a high fever. It is advised to take your temperature again after 30 minutes, pay attention to a body temperature change, and add a body temperature record. Would you like to enable a 30-minute scheduled reminder for temperature measurement?" The user interface 1140 further includes an "enable" control and an "ignore" control. The "enable" control is configured to trigger enabling of the temperature measurement reminder function. When the user performs an operation on the "enable" control, for example, a tap operation, the wearable device enables the temperature measurement reminder function in response to the tap operation. For example, the wearable device reminds, through vibration, a sound, a text, or the like, the user to perform body temperature measurement once at regular intervals (for example, 30 minutes), until a measured temperature decreases to a normal body temperature. The "ignore" control is configured to ignore the prompt information for enabling the temperature measurement reminder function.

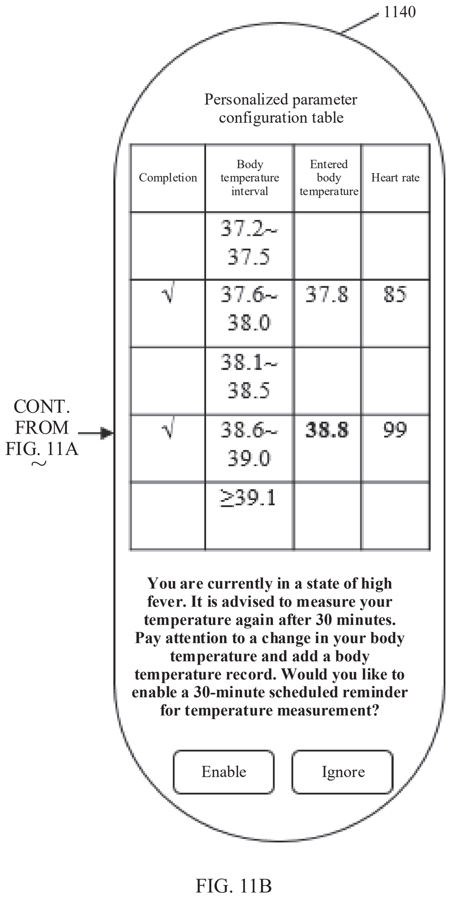

According to the example shown in FIG. 11A and FIG. 11B, the user may be reminded to pay more attention to a body temperature of the user and pay attention to a health problem. In addition, in a process in which the body temperature of the user gradually decreases from a high temperature to the normal body temperature, the user may be reminded for a plurality of times to perform body temperature measurement, to quickly obtain body temperature-heart rate data pairs in all ranges, and more quickly complete creation of the personalized body temperature model.

The following provides a body temperature measurement method applicable to the scenario 2.

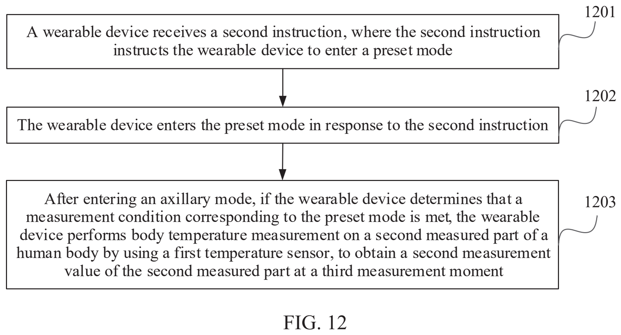

FIG. 12 is a schematic flowchart of a body temperature measurement method according to an embodiment of this application. The method may be performed by a wearable device or a chip or a component in the wearable device. The following embodiments are described by using an example in which the method is performed by the wearable device.

Step 1201: The wearable device receives a second instruction, where the second instruction instructs the wearable device to enter a preset mode.

The preset mode is a mode in which body temperature measurement is performed on a second measured part of a human body.

For example, the preset mode may include an axilla (or armpit) mode or a forehead mode. For example, when the preset mode is an axilla (or armpit) mode, the second measured part is an axilla (or armpit); or when the preset mode is a forehead mode, the second measured part is a forehead.

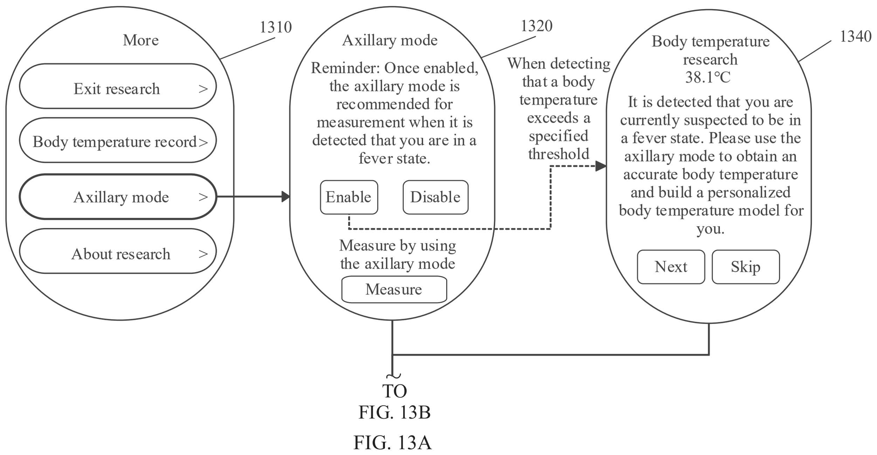

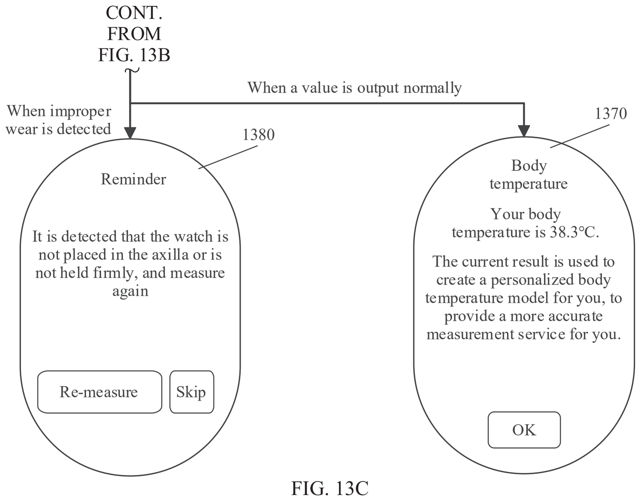

In some examples, that the preset mode is the axillary mode is used as an example. A user performs an operation on the wearable device, and the wearable device enters a user interface 1310 shown in FIG. 13A. The user interface 1310 includes an option of the axillary mode. When the user performs an operation, for example, a tap operation, on the option of the axillary mode, the wearable device displays a user interface 1320 in response to the tap operation. The user interface 1320 includes a measurement control used to trigger a first instruction. When the user performs an operation, for example, a tap operation on the measurement control, the wearable device displays a user interface 1330 in response to the tap operation. The user interface 1330 includes prompt information used to notify the user of a posture requirement for measuring a body temperature in the axillary mode. For example, the prompt information is as follows: "Sit down and place the watch in the axilla with a crown facing outward, ensuring the 9 o'clock direction is aligned with the axilla."

Optionally, the user interface 1320 may further include prompt information used to prompt the user to enable a reminder function of using the axillary mode. For example, the prompt information is as follows: "Reminder: Once enabled, the axillary mode is recommended for measurement when it is detected that you are in a fever state". The user interface 1320 may further include an enable control used to trigger enabling of the reminder function of using the axillary mode. When the user performs an operation on the enable control, for example, a tap operation, the wearable device enables the reminder function of using the axillary mode in response to the tap operation.

After the reminder function of using the axillary mode is enabled, in a process in which the user measures a body temperature by using the wearable device, when detecting that the body temperature exceeds a specified threshold, the wearable device prompts the user to measure the body temperature by using the axillary mode. For example, the wearable device detects that the body temperature of the human body is 38.1°C, and displays a user interface 1340. The user interface 1340 includes prompt information used to prompt the user to measure the body temperature by using the axillary mode. For example, the prompt information is as follows: "It is detected that you are currently suspected to be in a fever state. Use the axillary mode to obtain an accurate body temperature and establish a personalized body temperature model for you." The user interface 1340 may further include a next control, configured to trigger entering the axillary mode. When the user performs an operation on the next control, for example, a tap operation, the wearable device enters the axillary mode in response to the tap operation, and displays the user interface 1330. The user interface 1330 may further include a next control. When the user performs an operation on the next control, for example, a tap operation, the wearable device displays a user interface 1350 in response to the tap operation. The user interface 1350 may include information used to notify the user of measurement duration, a prompt manner of measurement completion, or the like.

Step 1202: The wearable device enters the preset mode in response to the second instruction.

Step 1203: After entering the axillary mode, if the wearable device determines that a measurement condition corresponding to the preset mode is met, the wearable device performs body temperature measurement on the second measured part of the human body by using a first temperature sensor, to obtain a second measurement value of the second measured part at a third measurement moment.

In a possible implementation, the wearable device obtains detection data of a motion sensor. The wearable device obtains a first temperature change rate corresponding to a plurality of third measurement values obtained by the first temperature sensor by performing body temperature measurement on the second measured part for a plurality of times within first preset duration after the wearable device enters the preset mode, and obtains a second temperature change rate corresponding to a plurality of fourth measurement values obtained by a second temperature sensor by performing body temperature measurement on the second measured part for a plurality of times within the first preset duration after the wearable device enters the preset mode. If the detection data meets a posture condition corresponding to the preset mode, the first temperature change rate is greater than or equal to a second threshold, and the second temperature change rate is greater than or equal to a third threshold, the wearable device determines that the measurement condition corresponding to the preset mode is met.

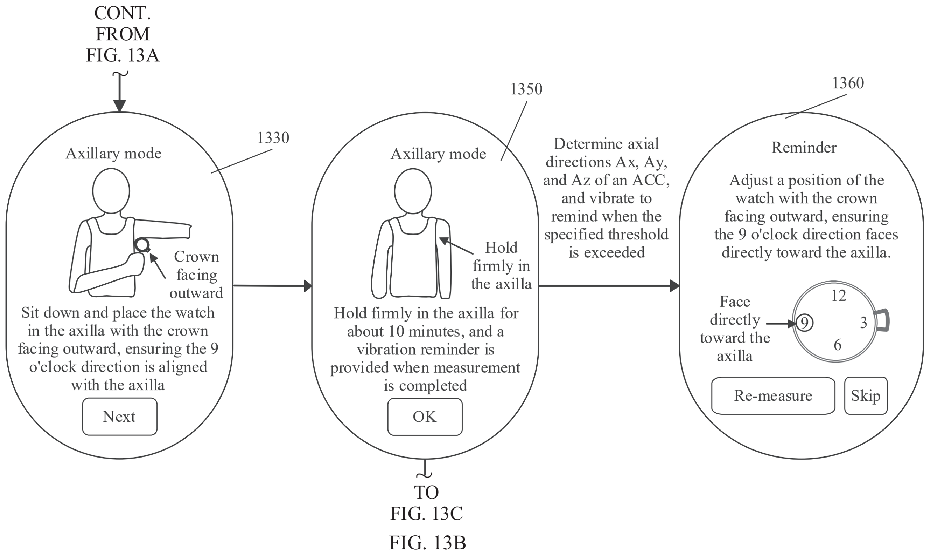

For example, the wearable device may determine whether a placement posture of the wearable device is correct by determining whether the detection data corresponding to the motion sensor meets the posture condition corresponding to the preset mode. For example, the motion sensor is an accelerometer (accelerometer, ACC). If axial directions Ax, Ay, and Az of the ACC exceed a specified threshold, that is, the detection data does not meet the posture condition corresponding to the preset mode, it indicates that the placement posture of the wearable device is incorrect, the wearable device may determine that the measurement condition corresponding to the preset mode is not met, and the wearable device may output third prompt information. The third prompt information is used to prompt to adjust the placement posture of the wearable device, or the third prompt information is used to prompt the user to adjust a position of the wearable device. Specific content of the third prompt information is not limited in this application. For example, prompt information in a user interface 1360 in FIG. 13B is as follows: "Adjust a position of the watch with a crown facing outward, ensuring the 9 o'clock direction faces directly toward the axilla". The user interface 1360 may further include a re-measurement control, and the re-measurement control is configured to perform body temperature measurement on the axilla part again.

If the axial directions Ax, Ay, and Az of the ACC do not exceed the specified threshold, it indicates that the detection data meets the posture condition corresponding to the preset mode, and it is determined that the placement posture of the wearable device is correct. Then, it may be determined, with reference to the first temperature change rate corresponding to the plurality of third measurement values obtained by the first temperature sensor through measurement within the first preset duration and the second temperature change rate corresponding to the plurality of fourth measurement values obtained by the second temperature sensor through measurement within the first preset duration, whether the wearable device is placed on the second measured part corresponding to the preset mode, to determine whether the wearable device meets the measurement condition corresponding to the preset mode.

For example, if the first temperature change rate is greater than or equal to the third threshold, and the second temperature change rate is greater than or equal to the fourth threshold, it is determined that the wearable device is placed on the second measured part of the human body. Therefore, the wearable device determines that the measurement condition corresponding to the preset mode is met.

Alternatively, if the first temperature change rate is less than the third threshold, or the second temperature change rate is less than the fourth threshold, it is determined that the wearable device is not placed on the second measured part of the human body. Therefore, the wearable device may determine that the measurement condition corresponding to the preset mode is not met, and the wearable device may output the third prompt information. The third prompt information is used to prompt the user to adjust the position of the wearable device. For example, the wearable device may remind, through vibration or voice, the user that the wearable device is not clamped in the axilla or the wearable device is not held firmly. The wearable device may further display a user interface 1380. The user interface 1380 may include prompt information used to prompt the user to perform measurement again. For example, the prompt information is as follows: "It is detected that the wearable device is not placed in the axilla or is not held firmly, and measure again".

When determining that the measurement condition corresponding to the preset mode is met, the wearable device may perform body temperature measurement on the second measured part of the human body by using the first temperature sensor, to obtain the second measurement value of the second measured part at the third measurement moment. In a possible implementation, after the wearable device determines that the measurement condition corresponding to the preset mode is met, if a fifth measurement value obtained by the first temperature sensor by performing body temperature measurement on the second measured part at a specific measurement moment and a sixth measurement value obtained by the second temperature sensor by performing body temperature measurement on the second measured part meet the preset condition, it indicates that temperature measurement is completed at a measurement moment that meets the preset condition. For example, the measurement moment that meets the preset condition is referred to as the third measurement moment, and the wearable device determines a measurement value obtained by the first temperature sensor by performing body temperature measurement on the second measured part at the third measurement moment as the second measurement value.

In some embodiments, after it is determined that the measurement condition corresponding to the preset mode is met, the wearable device may be limited to completing body temperature measurement within second preset duration after entering the preset mode. If the body temperature measurement is not completed within the second preset duration, the measurement ends. For example, the second preset duration is set to 10 minutes. If the wearable device completes temperature measurement within 10 minutes after entering the preset mode, a measurement result of the first temperature sensor when the temperature measurement is completed is used as a body temperature result of the human body. If the wearable device still fails to complete the temperature measurement 10 minutes after entering the preset mode, the user may be prompted to perform measurement again, or the measurement ends. Specific values of the first preset duration and the second preset duration are not limited in this application.

It should be understood that, after determining that the measurement condition corresponding to the preset mode is met, the wearable device may not be limited to completing body temperature measurement within the second preset duration. To be specific, provided that at any measurement moment after it is determined that the measurement condition corresponding to the preset mode is met, the fifth measurement value obtained by the first temperature sensor by performing body temperature measurement on the second measured part and the sixth measurement value obtained by the second temperature sensor by performing body temperature measurement on the second measured part meet the preset condition, it indicates that temperature measurement is completed at the measurement moment meeting the preset condition.

For example, the first temperature sensor in the wearable device measures that an axillary temperature of the user is 38.3°C, and the wearable device displays a user interface 1370. The user interface 1370 may include a body temperature value 38.3°C. The user interface 1370 may further include prompt information used to prompt the user to use the second measurement value to create a personalized body temperature model. For example, the prompt information is as follows: "The current result is used to create a personalized body temperature model for you, to provide a more accurate measurement service for you".

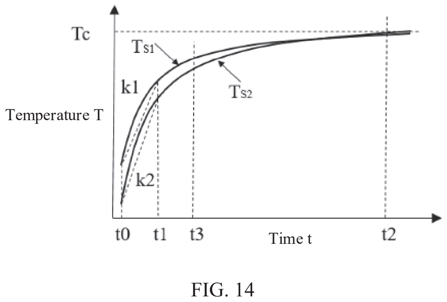

After it is determined that the wearable device meets the measurement condition corresponding to the preset mode, body temperature measurement is performed. For example, the wearable device is the smartwatch shown in FIG. 3, the first temperature sensor is the temperature sensor S1 in FIG. 3, and the second temperature sensor is the temperature sensor S2. When the wearable device is worn on a wrist, temperatures of the temperature sensor S1 and the temperature sensor S2 are low. After the wearable device is placed in the axilla, the temperature sensor S1 and the temperature sensor S2 are close to skin of the human body, causing temperature data obtained through measurement to rise rapidly. FIG. 14 is a diagram of a temperature change that is of a temperature sensor and that is obtained after a wearable device enters an axillary mode. As shown in FIG. 14, a curve T_{S1} is a curve in which a measured temperature of a temperature sensor S1 changes with time, and a curve T_{S2} is a curve in which a measured temperature of a temperature sensor S2 changes with time.

Based on FIG. 14, this application provides a schematic flowchart of a temperature measurement process after the wearable device enters the axillary mode.

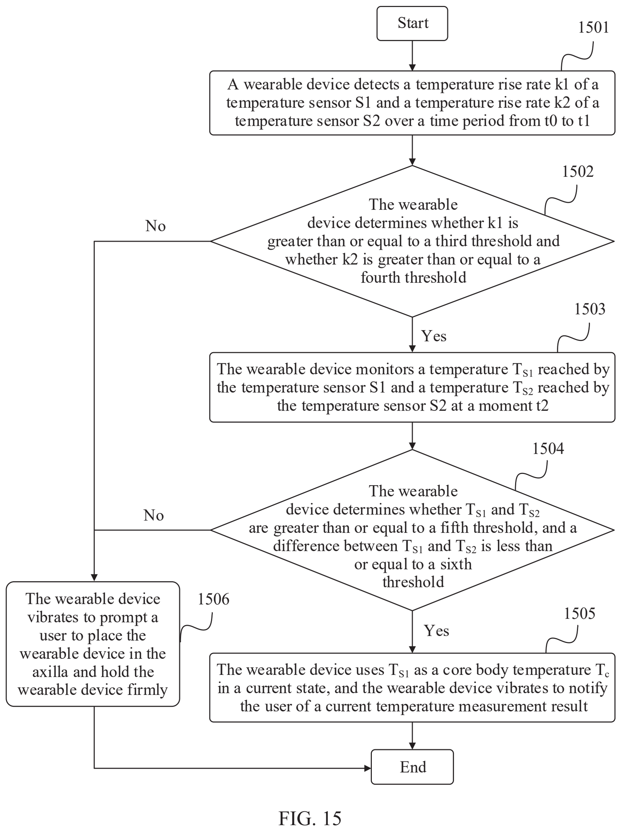

As shown in FIG. 15, the procedure includes the following steps.

Step 1501: The wearable device detects a temperature rise rate k1 of the temperature sensor S1 and a temperature rise rate k2 of the temperature sensor S2 over a time period from t0 to t1.

For example, with reference to FIG. 14, over the time period from t0 to t1, the temperature rise rate k1 of the temperature sensor S1 may be calculated based on the curve T_{S1}, and the temperature rise rate k2 of the temperature sensor S2 may be calculated based on the curve T_{S2}.

Step 1502: The wearable device determines whether k1 is greater than or equal to a third threshold and whether k2 is greater than or equal to a fourth threshold; and if k1 is greater than or equal to the third threshold and k2 is greater than or equal to the fourth threshold, performs step 1503; or if k1 is less than the third threshold or k2 is less than the fourth threshold, performs step 1506.

It is determined whether k1 is greater than or equal to the second threshold and whether k2 is greater than or equal to the third threshold, so that it may be determined whether a user clamps the wearable device in the axilla and holds the wearable device firmly. For example, the third threshold is k₁ₕ, and the fourth threshold is k₂ₕ. It is determined whether k1 is greater than or equal to k₁ₕ and whether k2 is greater than or equal to k₂ₕ. If it is determined that k1 is greater than or equal to k₁ₕ and k2 is greater than or equal to k₂ₕ, it indicates that the wearable device is clamped in the axilla and is held firmly, and step 1503 is performed. If it is determined that k1 is less than k₁ₕ, or k2 is less than k₂ₕ, it indicates that the wearable device is not clamped in the axilla or is not held firmly, and step 1506 is performed.

After it is determined that the wearable device is clamped in the axilla and is held firmly, temperatures of the temperature sensor S1 and the temperature sensor S2 rise exponentially over time, and finally reach a steady state at a moment t2. In addition, because the temperature sensor S2 is closer to the axilla, the temperature of S2 is generally slightly higher than that of S1. Whether temperature measurement is completed may be determined based on determining of whether temperature values of S1 and S2 exceed a set threshold and whether a difference between the temperature values is less than a set threshold.

Step 1503: The wearable device monitors the temperature T_{S1} reached by the temperature sensor S1 and the temperature T_{S2} reached by the temperature sensor S2 at the moment t2.

Step 1504: The wearable device determines whether the temperatures T_{S1} and T_{S2} are greater than or equal to a fifth threshold, and a difference between the temperatures T_{S1} and T_{S2} is less than or equal to a sixth threshold; and if the temperatures T_{S1} and T_{S2} are greater than or equal to the fifth threshold, and the difference between the temperatures T_{S1} and T_{S2} is less than or equal to the sixth threshold, performs step 1505; or if the temperatures T_{S1} and T_{S2} are less than the fifth threshold, or the difference between the temperatures T_{S1} and T_{S2} is greater than the sixth threshold, performs step 1506.

For example, the fifth threshold is T_{S1h}, and the sixth threshold is T_{dh}. The wearable device determines whether T_{S1} is greater than or equal to T_{S1h}, whether T_{S2} is greater than or equal to T_{S1h}, and whether abs(T_{S1}-T_{S2}) is less than or equal to T_{dh}. If it is determined that T_{S1} is greater than or equal to T_{S1h}, T_{S2} is greater than or equal to T_{S1h}, and abs(T_{S1}-T_{S2}) is less than or equal to T_{dh}, step 1505 is performed. If it is determined that T_{S1} is less than T_{S1h}, T_{S2} is less than T_{S1h}, or abs(T_{S1}-T_{S2}) is greater than T_{dh}, step 1506 is performed.

It should be understood that step 1504 may alternatively be replaced with the following: The wearable device determines whether the temperature T_{S1} is greater than or equal to a fifth threshold, and determines whether the temperature T_{S2} is greater than or equal to a seventh threshold, and a difference between the temperature T_{S1} and the temperature T_{S2} is less than or equal to a sixth threshold. For example, the fifth threshold is T_{S1h}, the sixth threshold is T_{dh}, and the seventh threshold is T_{S2h}. The wearable device determines whether T_{S1} is greater than or equal to T_{S1h}, whether T_{S2} is greater than or equal to T_{S2h}, and whether abs(T_{S1}-T_{S2}) is less than or equal to T_{dh}. If it is determined that T_{S1} is greater than or equal to T_{S1h}, T_{S2} is greater than or equal to T_{S2h}, and abs(T_{S1}-T_{S2}) is less than or equal to T_{dh}, step 1505 is performed. If it is determined that T_{S1} is less than T_{S1h}, T_{S2} is less than T_{S2h}, or abs(T_{S1}-T_{S2}) is greater than T_{dh}, step 1506 is performed.

Step 1505: The wearable device uses T_{S1} as a core body temperature Tc in a current state, and the wearable device vibrates to notify the user of a current temperature measurement result.

In some other embodiments, T_{S2} may alternatively be used as the core body temperature Tc in the current state, or an average value of T_{S1} and T_{S2} may be used as the core body temperature Tc in the current state. This is not limited in this application.

Step 1506: The wearable device vibrates to prompt the user to place the wearable device in the axilla and hold the wearable device firmly.

In some other embodiments, in a process from t0 to t2, the temperatures of the temperature sensor S1 and the temperature sensor S2 generally need about 10 minutes to reach a steady state. Therefore, the wearable device may alternatively predict steady-state axillary temperatures by using an algorithm based on the temperature rise rates of the temperature sensor S1 and the temperature sensor S2. In a possible implementation, the wearable device obtains a plurality of third measurement values obtained by a first temperature sensor by performing body temperature measurement on a second measured part for a plurality of times within first preset duration after the wearable device enters a preset mode and a measurement moment corresponding to each third measurement value, and obtains a plurality of fourth measurement values obtained by a second temperature sensor by performing body temperature measurement on the second measured part for a plurality of times within the first preset duration after the wearable device enters the preset mode and a measurement moment corresponding to each fourth measurement value. Then, a plurality of first sampling points and a plurality of second sampling points are obtained. Different first sampling points include different third measurement values and corresponding measurement moments, and different second sampling points include different fourth measurement values and corresponding measurement moments. The wearable device predicts, by using a least square method, a steady-state temperature value of the second measured part based on a first model, a second model, the plurality of first sampling points, and the plurality of second sampling points, to obtain a second measurement value. The first model is used to represent a relationship in which a temperature of the first temperature sensor changes over time within second preset duration, and the second model is used to represent a relationship in which a temperature of the second temperature sensor changes over time within the second preset duration. For example, the first preset duration is a time period from t0 to t3, and the second preset duration is a time period from t0 to t2. According to a transient heat conduction equation, the relationship between a temperature of the temperature sensor S1 and time has a simplified model (the first model) shown in the following Formula (5), and the relationship between a temperature of the temperature sensor S2 and time has a simplified model (the second model) shown in the following Formula (6): ${T}_{S 1} = Tc - \left(Tc-{T}_{S 10}\right) \times {e}^{- b 1 \times \left(t-t0\right)}$ ${T}_{S 2} = Tc - \left(Tc-{T}_{S 20}\right) \times {e}^{- b 2 \times \left(t-t0\right)}$

Herein, Tc is an axillary temperature, T_{S10} and T_{S20} are respectively temperatures of the temperature sensor S1 and the temperature sensor S2 at the initial moment t0, b1 and b2 are coefficients, and T_{S1} and T_{S2} are temperatures of the temperature sensor S1 and the temperature sensor S2 at a moment t.

Data sampling is performed a plurality of times in the time period from t0 to t3, to obtain the plurality of first sampling points, namely, (tₓᵢ, T_{S1xi}), where i = 1, 2, 3, ..., and the plurality of second sampling points, namely, (tₓᵢ, T_{S2xi}), where i = 1, 2, 3, ...., and then, an optimal solution of Tc may be obtained by using the least square method based on the foregoing Formula (5) and Formula (6), the plurality of first sampling points, and the plurality of second sampling points, as a body temperature measurement result, so that measurement time consumption can be reduced.

According to the foregoing embodiment, when there is no measurement tool like a mercury thermometer or an ear thermometer, the wearable device may be placed on the second measured part, and an accurate body temperature may also be obtained.

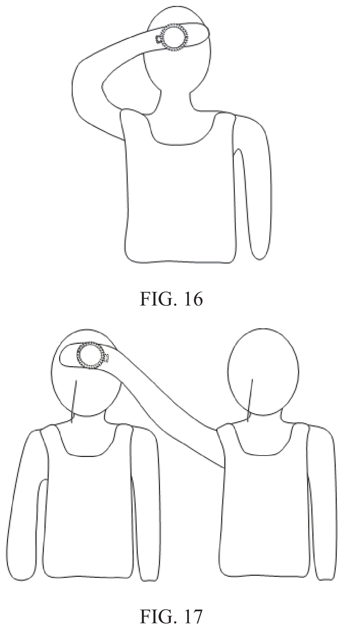

In the foregoing embodiment, the body temperature measurement method shown in FIG. 12 is described by using an example in which the preset mode is the axillary mode. It should be understood that the body temperature measurement method shown in FIG. 12 is also applicable to a forehead mode. For a specific method, refer to the body temperature measurement method corresponding to the axillary mode. Details are not described herein again. A difference between the axillary mode and the forehead mode lies in that a measurement posture in the axillary mode is different from that in the forehead mode. FIG. 16 is a diagram of a temperature measurement posture of a user for measuring a forehead temperature of the user according to an embodiment of this application. FIG. 17 is a diagram of a temperature measurement posture of a user for measuring a forehead temperature of another person according to an embodiment of this application.

When the preset mode in step 1201 is the forehead mode, with reference to the temperature measurement postures shown in FIG. 16 and FIG. 17, the first temperature sensor may be the temperature sensor S5 shown in FIG. 3. In this way, when wearing the wearable device on a wrist, the user may attach the back of a hand to a forehead of the user or another person. In this case, the temperature sensor S5 on a screen side is in contact with the forehead. When a temperature of the temperature sensor S5 reaches a stable state, the forehead temperature may be measured by using the temperature sensor S5, and a body temperature is obtained.

In the foregoing embodiments provided in this application, the methods provided in embodiments of this application are described from a perspective of the wearable device serving as an execution body. To implement functions in the foregoing methods provided in embodiments of this application, the wearable device may include a hardware structure and/or a software module, and the foregoing functions are implemented in a form of a hardware structure, a software module, or a combination of a hardware structure and a software module. Whether a function in the foregoing functions is performed by using the hardware structure, the software module, or the combination of the hardware structure and the software module depends on particular applications and design constraints of the technical solutions.

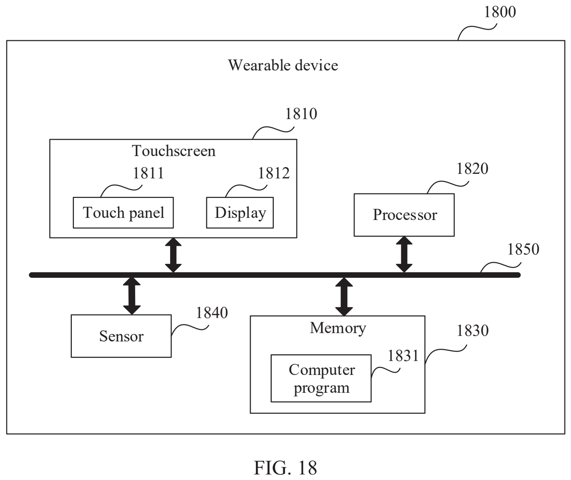

For example, when hardware is used for implementation, for hardware implementation of the wearable device, refer to FIG. 18 and related descriptions thereof.

Refer to FIG. 18. The wearable device may include: a touchscreen 1810, where the touchscreen 1810 includes a touch panel 1811 and a display 1812; one or more processors 1820; a memory 1830; one or more applications (not shown); one or more computer programs 1831; and one or more sensors 1840, for example, a first temperature sensor, a second temperature sensor, a motion sensor, and a heart rate sensor. The foregoing components may be connected through one or more communication buses 1850. The one or more computer programs 1831 are stored in the memory 1830 and are configured to be executed by the one or more processors 1820. The one or more computer programs 1831 include instructions, and the instructions may be used to perform the method in any one of the foregoing embodiments.

An embodiment of this application further provides a computer storage medium. The computer storage medium stores computer instructions. When the computer instructions are run on a wearable device, the wearable device is enabled to perform the foregoing related method steps to implement the methods in the foregoing embodiments.

An embodiment of this application further provides a computer program product. When the computer program product is run on a computer, the computer is enabled to perform the foregoing related steps to implement the methods in the foregoing embodiments.

In addition, an embodiment of this application further provides an apparatus. The apparatus may be specifically a chip, a component, or a module. The apparatus may include a processor and a memory that are connected. The memory is configured to store computer-executable instructions. When the apparatus runs, the processor may execute the computer-executable instructions stored in the memory, so that the chip performs the translation methods in the foregoing method embodiments.

The wearable device, the computer storage medium, the computer program product, or the chip provided in embodiments of this application is configured to perform the corresponding method provided above. Therefore, for beneficial effects that can be achieved by the wearable device, the computer storage medium, the computer program product, or the chip, refer to the beneficial effects in the corresponding method provided above. Details are not described herein again.

Based on the descriptions of the foregoing implementations, a person skilled in the art may understand that, for a purpose of convenient and brief description, division into the foregoing functional modules is merely used as an example for illustration. In actual application, the foregoing functions may be allocated to different functional modules for implementation based on a requirement. In other words, an inner structure of an apparatus is divided into different functional modules to implement all or some of the functions described above.

In the several embodiments provided in this application, it should be understood that the disclosed apparatus and method may be implemented in other manners. For example, the described apparatus embodiment is merely an example. For example, division into the modules or units is merely logical function division and may be other division in actual implementation. For example, a plurality of units or components may be combined or integrated into another apparatus, or some features may be ignored or not performed. In addition, the displayed or discussed mutual couplings or direct couplings or communication connections may be implemented through some interfaces. The indirect couplings or communication connections between the apparatuses or units may be implemented in an electrical form, a mechanical form, or another form.

The units described as separate parts may or may not be physically separate, and parts displayed as units may be one or more physical units, may be located in one place, or may be distributed on different places. Some or all of the units may be selected based on actual requirements to achieve the objectives of the solutions of embodiments.

In addition, functional units in embodiments of this application may be integrated into one processing unit, or each of the units may exist alone physically, or two or more units may be integrated into one unit. The integrated unit may be implemented in a form of hardware, or may be implemented in a form of a software functional unit.

When the integrated unit is implemented in the form of a software functional unit and sold or used as an independent product, the integrated unit may be stored in a readable storage medium. Based on such an understanding, the technical solutions of embodiments of this application essentially, or the part contributing to the conventional technology, or all or some of the technical solutions may be implemented in a form of a software product. The software product is stored in a storage medium and includes several instructions for instructing a device (which may be a single-chip microcomputer, a chip, or the like) or a processor (processor) to perform all or some of the steps of the methods described in embodiments of this application. The storage medium includes various media that can store program code, such as a USB flash drive, a removable hard disk, a read-only memory (read-only memory, ROM), a random access memory (random access memory, RAM), a magnetic disk, or an optical disc.

The foregoing descriptions are merely specific implementations of this application, but are not intended to limit the protection scope of this application. Any variation or replacement readily figured out by a person skilled in the art in the technical scope disclosed in this application shall fall within the protection scope of this application. Therefore, the protection scope of this application shall be subject to the protection scope of the claims.

## Claims

1. A body temperature measurement method, applied to a wearable device, wherein the wearable device comprises a first temperature sensor and a heart rate sensor, and the method comprises:
when the wearable device is worn on a human body, obtaining a first measurement value obtained by the first temperature sensor by performing body temperature measurement on a first measured part of the human body, wherein the first measurement value corresponds to a first measurement moment;
obtaining an ambient temperature at the first measurement moment and a first heart rate value obtained by the heart rate sensor by performing heart rate measurement on the human body at the first measurement moment;
inputting the first measurement value, and the ambient temperature and the first heart rate value at the first measurement moment into a body temperature model, to obtain a first body temperature value of the human body, wherein a structure parameter of the body temperature model is a preset value, or a structure parameter of the body temperature model is generated by performing model training based on standard body temperature values and heart rate values of the human body in historical duration; and
displaying the first body temperature value.

2. The method according to claim 1, wherein the structure parameter of the body temperature model is the preset value, and after obtaining the first body temperature value of the human body, the method further comprises:
displaying first prompt information if the first body temperature value is greater than or equal to a first threshold, wherein the first prompt information is used to prompt to enter the standard body temperature value.

3. The method according to claim 2, wherein after displaying the first prompt information, the method further comprises:
obtaining at least one entered second measurement moment and the standard body temperature value corresponding to each second measurement moment;
obtaining a second heart rate value obtained by the heart rate sensor through measurement at each second measurement moment;
obtaining at least one set of first data pairs, wherein different sets of first data pairs are created based on the standard body temperature values and the second heart rate values that correspond to different second measurement moments; and
updating the structure parameter of the body temperature model based on the at least one set of first data pairs.

4. The method according to claim 3, wherein updating the structure parameter of the body temperature model based on the at least one set of first data pairs comprises:
displaying second prompt information, wherein the second prompt information is used to prompt to update the structure parameter of the body temperature model;
receiving a first instruction, wherein the first instruction instructs the wearable device to update the body temperature model; and
updating, in response to the first instruction, the structure parameter of the body temperature model based on the at least one set of first data pairs.

5. The method according to claim 3 or 4, wherein before updating the structure parameter of the body temperature model based on the at least one set of first data pairs, the method further comprises:
determining that a quantity of data pairs in the at least one set of first data pairs reaches a quantity threshold.

6. The method according to claim 3 or 4, wherein after updating the structure parameter of the body temperature model, the method further comprises:
updating the structure parameter of the body temperature model when the wearable device creates a first data pair or deletes a created first data pair.

7. The method according to any one of claims 2 to 6, wherein the method further comprises:
if the first body temperature value is greater than or equal to a second threshold, prompting to enable a scheduled temperature measurement reminder function, wherein the second threshold is greater than the first threshold.

8. The method according to any one of claims 1 to 7, wherein the method further comprises:
receiving a second instruction, wherein the second instruction instructs the wearable device to enter a preset mode, and the preset mode is a mode in which body temperature measurement is performed on a second measured part of the human body;
after entering the preset mode in response to the second instruction, if it is determined that the wearable device meets a measurement condition corresponding to the preset mode, performing body temperature measurement on the second measured part of the human body by using the first temperature sensor and/or a second temperature sensor, to obtain a second measurement value of the second measured part at a third measurement moment; and
displaying the second measurement value.

9. The method according to claim 8, wherein the second measured part comprises an axilla or a forehead.

10. The method according to claim 8 or 9, wherein the wearable device further comprises the second temperature sensor and a motion sensor; and
determining that the wearable device meets the measurement condition corresponding to the preset mode comprises:
obtaining detection data of the motion sensor;
obtaining a first temperature change rate corresponding to a plurality of third measurement values obtained by the first temperature sensor by performing body temperature measurement on the second measured part for a plurality of times within first preset duration after the wearable device enters the preset mode, and obtaining a second temperature change rate corresponding to a plurality of fourth measurement values obtained by the second temperature sensor by performing body temperature measurement on the second measured part for a plurality of times within the first preset duration after the wearable device enters the preset mode; and
if the detection data meets a posture condition corresponding to the preset mode, the first temperature change rate is greater than or equal to a third threshold, and the second temperature change rate is greater than or equal to a fourth threshold, determining that the wearable device meets the measurement condition corresponding to the preset mode.

11. The method according to any one of claims 6 to 10, wherein the method further comprises:
if it is determined that the wearable device does not meet the measurement condition corresponding to the preset mode, displaying third prompt information, wherein the third prompt information is used to prompt to adjust a placement posture of the wearable device.

12. The method according to any one of claims 8 to 11, wherein the third measurement moment is a corresponding measurement moment when a fifth measurement value obtained by the first temperature sensor by performing body temperature measurement on the second measured part and a sixth measurement value obtained by the second temperature sensor by performing body temperature measurement on the second measured part meet a preset condition; and
the preset condition comprises: the fifth measurement value is greater than or equal to a fifth threshold, the sixth measurement value is greater than or equal to the fifth threshold, and a difference between the fifth measurement value and the sixth measurement value is less than or equal to a sixth threshold.

13. The method according to claim 12, wherein performing body temperature measurement on the second measured part of the human body by using the first temperature sensor and/or the second temperature sensor, to obtain the second measurement value of the second measured part at the third measurement moment comprises:
using the fifth measurement value or the sixth measurement value as the second measurement value, or
using an average value of the fifth measurement value and the sixth measurement value as the second measurement value.

14. The method according to claim 12, wherein the method further comprises:
obtaining the plurality of third measurement values obtained by the first temperature sensor by performing body temperature measurement on the second measured part for the plurality of times within the first preset duration after the wearable device enters the preset mode and a measurement moment corresponding to each third measurement value, and obtaining the plurality of fourth measurement values obtained by the second temperature sensor by performing body temperature measurement on the second measured part for the plurality of times within the first preset duration after the wearable device enters the preset mode and a measurement moment corresponding to each fourth measurement value;
obtaining a plurality of first sampling points and a plurality of second sampling points, wherein different first sampling points comprise different third measurement values and corresponding measurement moments, and different second sampling points comprise different fourth measurement values and corresponding measurement moments; and
obtaining the second measurement value through calculation by using a least square method based on a first model, a second model, the plurality of first sampling points, and the plurality of second sampling points, wherein the first model is used to represent a relationship in which a temperature of the first temperature sensor changes over time within second preset duration, and the second model is used to represent a relationship in which a temperature of the second temperature sensor changes over time within the second preset duration.

15. The method according to any one of claims 8 to 14, wherein after obtaining the second measurement value of the second measured part at the third measurement moment, the method further comprises:
obtaining a third heart rate value obtained by the heart rate sensor by performing heart rate measurement on the human body at the third measurement moment; and
creating a second data pair based on the second measurement value and the third heart rate value, wherein the second data pair is used to update the structure parameter of the temperature model.

16. A wearable device, comprising a processor, a memory, a temperature sensor, a heart rate sensor, and a display, wherein
the display is configured to display a user interface;
the temperature sensor is configured to perform body temperature measurement on a first measured part or a second measured part of a human body;
the heart rate sensor is configured to perform heart rate measurement on the human body; the memory is configured to store one or more computer programs; and
the processor is configured to: execute the one or more computer programs, and perform the method according to any one of claims 1 to 15 with reference to data obtained by the temperature sensor through measurement and data obtained by the heart rate sensor through measurement.

17. The wearable device according to claim 16, wherein the wearable device further comprises a motion sensor, configured to detect a placement posture of the wearable device.

18. A computer-readable storage medium, comprising a program or instructions, wherein when the program or the instructions are run on a computer, the method according to any one of claims 1 to 15 is performed.

19. A computer program product, wherein the computer program product stores a computer program, the computer program comprises program instructions, and when the program instructions are executed by a computer, the computer is enabled to perform the method according to any one of claims 1 to 15.
